# EUROPÄISCHE PATENTSCHRIFT

(11) **EP 0 459 949 B1**
(45) Veröffentlichungstag und Bekanntmachung des Hinweises auf die Patenterteilung: **03.01.1996**
(21) Anmeldenummer: 91810387.0
(22) Anmeldetag: 22.05.1991
(51) Int. Cl.: C07D 401/12

(54) **Sulfonylharnstoffe mit herbizider und pflanzenwuchsregulierender Wirkung**
Sulfonyl ureas with herbicidal activity and useful as plant growth regulators
Sulfonyl-urées avec activité herbicide et utiles comme regulateurs de la croissance des plantes

(30) Priorität: 30.05.1990 CH 1826/90
(43) Veröffentlichungstag der Anmeldung: 04.12.1991
(73) Patentinhaber: CIBA-GEIGY AG, CH-4002 Basel (CH)
(72) Erfinder: Föry, Werner, Dr., CH-4125 Riehen (CH); Schurter, Rolf, Dr., CH-4102 Binningen (CH)

(56) Entgegenhaltungen:
- EP-A- 0 103 543
- EP-A- 0 165 753
- US-A- 4 522 645
- US-A- 4 657 578
- US-A- 4 707 179
- US-A- 4 746 353
- US-A- 4 789 465
- CHEMICAL ABSTRACTS, Band 102, Nr. 7, 18. Februar 1985, Columbus, Ohio, USA R. LEJEUNE et al. "Preparation of the disulfide of 3-mercaptopyridine-2-sulfonamide." Seite 582, Spalte 1, Zusammenfassung-Nr. 62 050z

## Beschreibung

Die vorliegende Erfindung betrifft neue, herbizid wirksame und pflanzenwuchsregulierende N-Pyridinsulfonyl-N'-pyrimidinyl- und -triazinylharnstoffe, Verfahren zu ihrer Herstellung, sie als Wirkstoffe enthaltende Mittel, sowie deren Verwendung zum Bekämpfen von Unkräutern, vor allem selektiv in Nutzpflanzenkulturen oder zum Regulieren und Hemmen des Pflanzenwachstums.

Harnstoffverbindungen, Triazinverbindungen und Pyrimidinverbindungen mit herbizider Wirkung sind allgemein bekannt. Beispielsweise beschreibt das Europäische Patent Nr. 103 543 sowie das U.S. Patent Nr. 4,544,401 herbizid wirksame und pflanzenwuchsregulierende N-Pyridinsulfonyl-N'-pyrimidinyl- und -triazinylharnstoffe. Die dort offenbarten Wirkstoffe können jedoch die Anforderungen bezüglich Wirkungsstärke und Selektivität nicht immer erfüllen. Es besteht somit ein Bedarf nach besser wirksamen und selektiveren Wirkstoffen.

CAS 102(7), Nr. 62050Z (1985) beschreibt Pyridinsulfonamide der Formel II, worin R₁ und R₂ Wasserstoff sind.

Es wurden nun neue Sulfonylharnstoffe mit verbesserten herbiziden und pflanzenwachstumsregulierenden Eigenschaften gefunden.

Die erfindungsgemäßen N-Pyridinsulfonyl-N'-pyrimidinyl- und -triazinylharnstoffe entsprechen der Formel I 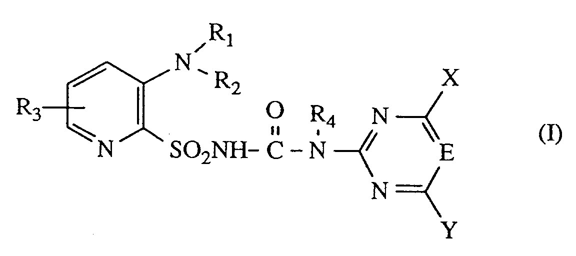
worin
R₁ und R₂ unabhängig voneinander Wasserstoff, C₁-C₈-Alkyl oder C₃-C₈-Cycloalkyl; durch Halogen ein- oder mehrfach substituiertes C₂-C₈-Alkyl oder C₃-C₈-Cycloalkyl; durch -NR₅R₆, C₁-C₄-Alkoxy oder C₁-C₄-Alkyl-S(O)ₙ- substituiertes C₁-C₈-Alkyl oder C₃-C₈-Cycloalkyl; durch C₂-C₄-Alkenyl, C₅-C₆-Cycloalkenyl, C₂-C₆-Alkinyl oder C₄-C₈-Alkadienyl substituiertes C₁-C₄-Alkyl oder C₃-C₆-Cycloalkyl; wobei die C₂-C₄-Alkenyl-, C₅-C₆-Cycloalkenyl- und C₄-C₈-Alkadienylreste durch Halogen ein- oder mehrfach substituiert sein können; oder R₁ und R₂ zusammen eine 4-5-gliedrige C₄-C₁₀-Alkylenkette, die durch Sauerstoff, Schwefel oder N-R₇ unterbrochen sein kann;
R₃ Wasserstoff, Halogen, C₁-C₄-Alkyl, C₁-C₄-Alkoxy oder C₁-C₄-Alkylthio; durch Halogen ein- oder mehrfach substituiertes C₁-C₄-Alkyl, C₁-C₄-Alkoxy oder C₁-C₄-Alkylthio;
R₄, R₅, R₆ und R₇ unabhängig voneinander Wasserstoff oder C₁-C₄-Alkyl;
n 0, 1 oder 2;
X C₁-C₃-Alkyl, durch Halogen ein- bis dreifach substituiertes C₁-C₃-Alkyl;
C₁-C₃-Alkoxy oder durch Halogen ein- bis dreifach substituiertes C₁-C₃-Alkoxy;
Y Halogen, C₁-C₃-Alkyl, durch Halogen ein- bis dreifach substituiertes C₁-C₃-Alkyl;
C₁-C₃-Alkoxy, durch Halogen ein- bis dreifach substituiertes C₁-C₃-Alkoxy; Cyclopropyl, Methylamino oder Dimethylamino; und
E Stickstoff oder die Methingruppe bedeuten,
sowie die Salze dieser Verbindungen.

Die als oder in den Substituenten R₁bis R₇ vorkommenden Alkylgruppen können geradkettig oder verzweigt sein und stehen beispielsweise für Methyl, Ethyl, n-Propyl, iso-Propyl, n-Butyl, sek.-Butyl, iso-Butyl, tert.-Butyl, n-Pentyl oder die isomeren Pentyl, n-Hexyl oder die isomeren Hexyl, n-Heptyl oder die isomeren Heptyl, sowie n-Oktyl oder die isomeren Oktyl. Vorzugsweise besitzen die als oder in den Substituenten vorkommendenen Alkylgruppen 1-4 Kohlenstoffatome.

Die als oder in den Substituenten R₁-R₃ vorkommenden ein- oder mehrfach durch Halogen substituierte Alkylgruppen umfassen geradkettige oder verzweigte Alkylgruppen wie Methyl, Ethyl, n-Propyl, iso-Propyl, n-Butyl, sek.-Butyl, iso-Butyl, tert.-Butyl, n-Pentyl, die isomeren Pentyl, n-Hexyl sowie die isomeren Hexyl, n-Heptyl oder die isomeren Heptyl, sowie n-Oktyl oder die isomeren Oktyl, die ein- oder mehrfach durch Halogen substituiert sind, wobei Halogen im einzelnen Fluor, Chlor, Brom oder Jod bedeutet.

Unter diesen ein- oder mehrfach durch Halogen substituierten Alkylgruppen sind ein- bis dreifach durch Halogen substituierte Alkylgruppen bevorzugt. Bevorzugte Halogenatome, die als Substituenten der Alkylgruppen auftreten können, sind Fluor und Chlor. Speziell bevorzugte ein- oder mehrfach durch Halogen substituierte Alkylgruppen sind Trifluormethyl, 1-Fluorethyl, 1,1-Dichlorethyl, 3,3,3-Trifluorpropyl, 2-Fluorisopropyl, 3-Fluorpropyl, 1,1,1-Trichlorpentyl, 1-Fluor-3-methylpentyl, oder 1-Bromhexyl. Ganz besonders bevorzugt sind 3-Fluorpropyl und 2-Fluorisopropyl.

Die als oder in den Substituenten X und Y vorkommenden C₁-C₃-Alkylreste umfassen im einzelnen Methyl, Ethyl, n-Propyl und iso-Propyl sowie die von diesen Resten abgeleiteten ein- bis dreifach durch Halogen substituierten Halogenalkyle. Vorzugsweise besitzen die als oder in den Substituenten X und Y vorkommenden Alkylreste ein bis zwei Kohlenstoffatome.

Bei den als oder in den Substituenten X und Y vorkommenden ein- bis dreifach durch Halogen substituierten C₁-C₃-Alkylgruppen sind ein- bis dreifach durch Fluor oder Chlor substituierte C₁-C₂-Alkylgruppen bevorzugt. Speziell bevorzugte, als oder in den Substituenten X und Y vorkommende, ein- bis dreifach durch Halogen substituierte C₁-C₃-Alkylreste sind: Trifluormethyl, Difluormethyl, 2-Chlorethyl, Chlordifluormethyl, Dichlormethyl, Chlorfluormethyl, 1,1-Dichlorethyl, Trifluorethyl, 3,3,3-Trifluorpropyl oder 2,3-Dichlorpropyl, besonders bevorzugt sind Fluormethyl, Chlormethyl, Difluormethyl und Trifluormethyl.

Die in den Substituenten R₁ und R₂ vorkommenden C₂-C₄-Alkenylreste können in der Z-Form (cis) oder in der E-Form (trans) vorliegen und können geradkettig oder verzweigt sein. Bevorzugt sind Alkenylreste mit einer Kettenlänge von zwei bis drei Kohlenstoffatomen. Beispiele für C₂-C₄-Alkenylreste sind: Vinyl, Allyl, Methallyl, 1-Methylvinyl, But-2-en-1-yl. Bevorzugt sind Vinyl und Allyl. Bei den C₂-C₄-Alkenylresten, welche ein- oder mehrfach durch Halogen substituiert sind, steht Halogen im einzelnen für Fluor, Chlor, Brom und Jod. Bevorzugte Halogenatome, die als Substituenten der C₂-C₄-Alkenylreste auftreten können, sind Fluor und Chlor. Unter den durch Halogen ein- bis dreifach substituierten C₂-C₄-Alkenylresten sind diejenigen bevorzugt, die eine Kettenlänge von zwei bis drei Kohlenstoffatomen besitzen. Speziell bevorzugte, durch Halogen ein- bis dreifach substituierte C₂-C₄-Alkenylreste sind 1-Chlorvinyl, 2-Chlorvinyl, 3-Fluorallyl und 4,4,4-Trifluor-but-2-en-1-yl. Ganz besonders bevorzugt sind 1-Chlorvinyl und 2-Chlorvinyl.

Die in den Substituenten R₁ und R₂ vorkommenden C₄-C₈-Alkadienylreste, die durch Halogen ein- oder mehrfach substituiert sein können, stehen beispielsweise für But-1,3-dienyl, Pent-1,3-dienyl, Hex-3,5-dienyl, Hept-4,6-dienyl, Okt-1,7-dienyl, 4-Chlor-but-1,3-dienyl, oder 5,5,5,-Trifluor-pent-1,3-dienyl.

Die in den Definitionen der Substituenten R₁ und R₂ vorkommenden C₂-C₆-Alkinylreste können geradkettig oder verzweigt sein. Bevorzugt sind Alkinylreste mit einer Kettenlänge von 2 bis 3 Kohlenstoffatomen. C₂-C₄-Alkinylreste stehen beispielsweise für Ethinyl, Propargyl, 1-Propinyl, 3-Butinyl, 1-Methylpropargyl, 3-Pentinyl oder 3-Hexinyl, wobei Ethinyl und Propargyl besonders bevorzugt sind.

Die in den Substituenten R₁ und R₂ vorkommenden unsubstituierten oder durch Halogen ein- oder mehrfach substituierte Cycloalkylgruppen umfassen beispielsweise Cyclopropyl, Cyclobutyl, Cyclopentyl, Cyclohexyl, Cycloheptyl, Cyclooctyl, 2-Fluorcyclopropyl, 2,3-Difluorcyclopropyl, 2,2-Dichlorcyclopropyl, 3-Bromcyclopropyl, 2,3,4-Trifluorcyclopentyl oder 2,3-Dichlorcyclohexyl. Von diesen Cycloalkylgruppen sind die ein- bis zweifach durch Halogen substituierte Cyclopropyl-, Cyclopentyl- und Cyclohexylgruppen bevorzugt wobei Halogen für Fluor, Chlor, Brom und Jod, insbesondere aber für Fluor und Chlor steht.

Weitere substituierte Cycloalkylgruppen sind beispielsweise 2-Methoxycyclopropyl, 3-Ethylthiocyclobutyl, 2-Methylsulfonylcyclopentyl, 3-Ethylsulfinylcyclohexyl, 4-(2-4-pentadienyl)-cyclohexan, 3-Amino-cyclohexyl, 4-Allyl-cyclohexyl oder 3-Propargylcyclohexyl.

Die C₅-C₆-Cycloalkenylreste können ein- bis dreifach durch Halogen substituiert sein. Bevorzugte Halogenatome sind dabei Fluor und Chlor. Beispiele für C₅-C₆-Cycloalkenylreste sind 3-Cyclopenten, 2-Cyclopenten, 4-Chlor-cyclopent-3-en, 3,4-Difluorcyclopent-3-en, 2-Cyclohexen, 3-Cyclohexen, 4,5-Dibrom-cyclohex-2-en oder 4,4,5-Trifluorcyclohex-2-en.

Beispiele für Heterozyklen, die die Substituenten R₁ und R₂ zusammen mit dem Stickstoffatom, an welches sie gebunden sind, bilden können sind: Pyrrolidin, 3-Methylpyrrolidin, Imidazolidin, Piperidin, 3-Isopropyl-piperidin, Piperazin, 4-Methylpiperazin, 4-Ethylpiperazin, 4-Isopropylpiperazin, Morpholin, Oxazolidin, und Thiazolidin. Vorzugsweise bilden die Substituenten R₁ und R₂ 4-5-gliedrige C₄-C₅-Alkylenketten. Bevorzugte Heterozyklen, die die Substituenten R₁ und R₂ zusammen mit dem Stickstoffatom, an welches sie gebunden sind, bilden können, sind: 4-Methylpiperazin, Morpholin und Piperidin.

Alkylthio ist beispielsweise Methylthio, Ethylthio, Propylthio, Isopropylthio, n-Butylthio, i-Butylthio, s-Butylthio, t-Butylthio oder die isomeren Pentylthio, vorzugsweise Methylthio und Ethylthio.

Alkoxy ist beispielsweise Methoxy, Ethoxy, Propyloxy, i-Propyloxy, n-Butyloxy, i-Butyloxy, s-Butyloxy und t-Butyloxy; vorzugsweise Methoxy und Ethoxy.

Die Erfindung umfaßt ebenfalls die Salze, die die Verbindungen der Formel I mit Aminen, Alkali- und Erdalkalimetallbasen oder quaternären Ammoniumbasen bilden können.

Unter Alkali- und Erdalkalimetallhydroxiden als Salzbildner sind die Hydroxide von Lithium, Natrium, Kalium, Magnesium oder Calcium hervorzuheben, insbesondere aber die von Natrium oder Kalium.

Beispiele für zur Salzbildung geeignete Amine sind primäre, sekundäre und tertiäre aliphatische und aromatische Amine wie Methylamin, Ethylamin, Propylamin, iso-Propylamin, die vier isomeren Butylamine, Dimethylamin, Diethylamin, Diethanolamin, Dipropylamin, Diisopropylamin, Di-n-butylamin, Pyrrolidin, Piperidin, Morpholin, Trimethylamin, Triethylamin, Tripropylamin, Chinuclidin, Pyridin, Chinolin und iso-Chinolin, insbesondere aber Ethyl-, Propyl-, Diethyl- oder Triethylamin, vor allem aber iso-Propylamin und Diethanolamin.

Beispiele für quaternäre Ammoniumbasen sind im allgemeinen die Kationen von Halogenammoniumsalzen, z.B. das Tetramethylammoniumkation, das Trimethylbenzylammoniumkation, das Triethylbenzylammoniumkation, das Tetraethylammoniumkation, das Trimethylethylammoniumkation, aber auch das Ammoniumkation.

Aus dem Umfang der Formel I sind diejenigen Verbindungen hervorzuheben, in denen R₁ und R₂ unabhängig voneinander Wasserstoff, C₁-C₈-Alkyl oder C₃-C₈-Cycloalkyl; durch Halogen ein- oder mehrfach substituiertes C₂-C₈-Alkyl oder C₃-C₈-Cycloalkyl; durch -NR₅R₆, C₁-C₄-Alkoxy oder C₁-C₄-Alkyl-S(O)ₙ- substituiertes C₁-C₈-Alkyl oder C₃-C₈-Cycloalkyl; durch C₂-C₄-Alkenyl, C₅-C₆-Cycloalkenyl, C₂-C₆-Alkinyl oder C₄-C₈-Alkadienyl substituiertes C₁-C₄-Alkyl oder C₃-C₆-Cycloalkyl; wobei die C₂-C₄-Alkenyl-, C₅-C₆-Cycloalkenyl- und C₄-C₈-Alkadienylreste durch Halogen ein- oder mehrfach substituiert sein können; bedeuten.

Unter den Verbindungen der Formel I sind ferner diejenigen bevorzugt, in denen R₁ und R₂ unabhängig voneinander Wasserstoff, C₁-C₆-Alkyl oder C₃-C₆-Cycloalkyl; durch Halogen ein- bis dreifach substituiertes C₂-C₆-Alkyl oder C₃-C₆-Cycloalkyl; durch -NR₅R₆, C₁-C₄-Alkoxy oder C₁-C₄-Alkyl-S(O)ₙ- substituiertes C₁-C₆-Alkyl oder C₃-C₆-Cycloalkyl; durch C₂-C₄-Alkenyl, C₅-C₆-Cycloalkenyl, C₂-C₄-Alkinyl oder C₄-C₈-Alkadienyl substituiertes C₁-C₄-Alkyl oder C₃-C₆-Cycloalkyl; wobei die C₂-C₄-Alkenyl-, C₅-C₆-Cycloalkenyl- und C₄-C₈-Alkadienylreste durch Halogen ein- bis dreifach substituiert sein können; und
R₃ Wasserstoff, Halogen, C₁-C₄-Alkyl, C₁-C₄-Alkoxy oder C₁-C₄-Alkylthio; oder durch Halogen ein- bis dreifach substituiertes C₁-C₄-Alkyl, C₁-C₄-Alkoxy oder C₁-C₄-Alkylthio bedeutet.

Ferner sind unter den Verbindungen der Formel I diejenigen von besonderem Interesse, in denen
mindestens einer der Substituenten R₁, R₂ und R₃ verschieden von Wasserstoff ist.

Ganz besonders bevorzugte Gruppen von Verbindungen der Formel I sind jene, in welchen
a) X für C₁-C₃-Alkyl, C₁-C₃-Alkoxy oder durch Halogen ein- bis dreifach substituiertes C₁-C₃-Alkoxy; und
   Y für Chlor, C₁-C₃-Alkyl, C₁-C₃-Alkoxy oder durch Halogen ein- bis dreifach substituiertes C₁-C₃-Alkoxy steht; oder
b) X für Methyl, Methoxy, Ethoxy oder Difluormethoxy; und
   Y für Methyl, Methoxy, Ethoxy, Difluormethoxy oder Chlor steht; oder
c) X für Methoxy oder Ethoxy; und Y für Methyl oder Methoxy steht;
d) E die Methinbrücke bedeutet; oder
e) R₃ in der 6-Stellung des Pyridinringes steht; oder
f) R₃ Wasserstoff bedeutet.

Von diesen Verbindungen sind diejenigen besonders hervorzuheben, in denen
a) R₂ und R₃ für Wasserstoff und R₁ für C₁-C₆-Alkyl oder C₃-C₆-Cycloalkyl; durch Halogen ein- bis dreifach substituiertes C₂-C₆-Alkyl oder C₃-C₆-Cycloalkyl; durch -NR₅R₆, C₁-C₄-Alkoxy oder C₁-C₄-Alkyl-S(O)ₙ- substituiertes C₁-C₆-Alkyl oder C₃-C₆-Cycloalkyl; durch C₂-C₄-Alkenyl, C₅-C₆-Cycloalkenyl, C₂-C₄-Alkinyl oder C₄-C₈-Alkadienyl substituiertes C₁-C₄-Alkyl oder C₃-C₆-Cycloalkyl; wobei die C₂-C₄-Alkenyl-, C₅-C₆-Cycloalkenyl- und C₄-C₈-Alkadienylreste durch Halogen ein- bis dreifach substituiert sein können; steht; oder
b) R₁ C₁-C₆-Alkyl, Allyl oder Propargyl und R₂ Wasserstoff, Methyl oder Allyl bedeutet.

Als bevorzugte Einzelverbindung aus dem Umfang der Formel I ist zu nennen: N-(3-n-Butylamino-pyridin-2-yl-sulfonyl)-N'-(4,6-dimethoxypyrimidin-2yl)-harnstoff.

Die Verbindungen der Formel I können hergestellt werden, indem man
a) ein Pyridylsulfonamid der Formel II 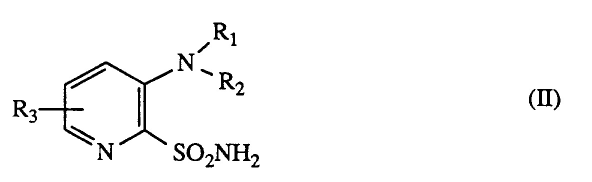 worin R₁, R₂ und R₃ die unter Formel I im Anspruch 1 angegebene Bedeutung haben, mit einem N-Pyrimidinyl- oder N-Triazinylcarbamat der Formel III 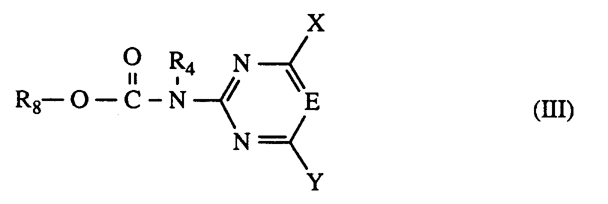 worin X, Y, R₄ und E die unter Formel I in Anspruch 1 angegebene Bedeutung haben und R₈ C₁-C₄-Alkyl, oder Phenyl, welches durch C₁-C₄-Alkyl oder Halogen substituiert sein kann, bedeutet; in Gegenwart einer Base umsetzt, oder
b) ein Pyridylsulfonamid der Formel XII

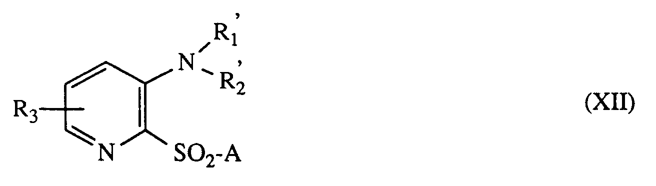

worin R₁', R₂' die unter R₁ und R₂ in Formel I im Anspruch 1 angegebene Bedeutung mit Ausnahme von Wasserstoff haben, R₃ die unter Formel I im Anspruch 1 angegebene Bedeutung hat und A 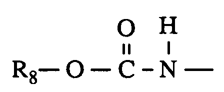
oder O=C=N- bedeutet, wobei R₈ die oben angegebene Bedeutung hat, in Gegenwart einer Base mit einem 2-Aminopyrimidin- oder -triazin der Formel XI 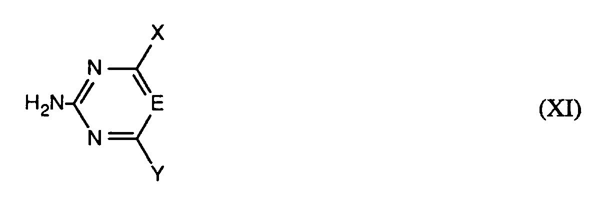
worin E, X und Y die unter Formel I angegebene Bedeutung haben, umsetzt.

Verbindungen der Formel Ia 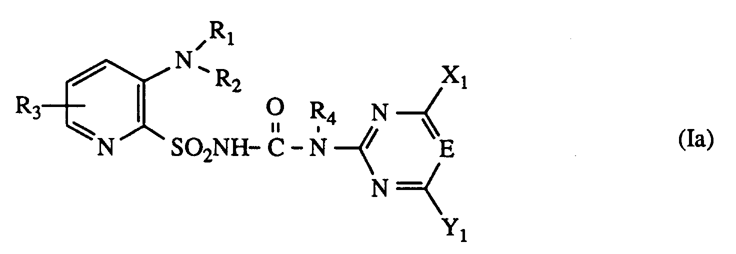
worin R₁, R₂, R₃, R₄ und E die unter Formel I angegebene Bedeutung haben und X₁ und Y₁ unabhängig voneinander für Chlor oder Difluormethoxy stehen, können hergestellt werden, indem man ein Pyridylsulfonamid der Formel II 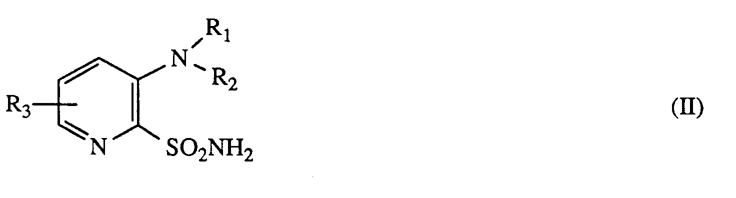
worin R₁, R₂ und R₃ die unter Formel I im Anspruch 1 angegebene Bedeutung haben, in Gegenwart einer Base mit einem Pyrimidinyl- oder Triazinylisocyanat der Formel IV 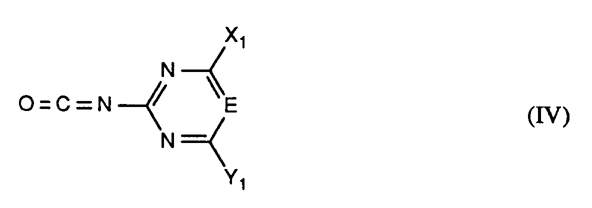
worin X₁ und Y₁ die unter Formel Ia angegebene Bedeutung haben und E die unter Formel I angegebene Bedeutung hat, umsetzt.

Die Umsetzungen zu Verbindungen der Formel I werden vorteilhafterweise in aprotischen, inerten organischen Lösungsmitteln vorgenommen. Solche Lösungsmittel sind Kohlenwasserstoffe wie Benzol, Toluol, Xylol oder Cyclohexan, chlorierte Kohlenwasserstoffe wie Dichlormethan, Trichlormethan, Tetrachlormethan oder Chlorbenzol, Ether wie Diethylether, Ethylenglykoldimethylether, Diethylenglykoldimethylether, Tetrahydrofuran oder Dioxan, Nitrile wie Acetonitril oder Propionitril, Amide wie Dimethylformamid, Diethylformamid oder N-Methylpyrrolidinon. Die Reaktionstemperaturen liegen vorzugsweise zwischen -20° und +120°C.
Die Umsetzungen verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können ebenfalls durch Zugabe einiger Tropfen Base als Reaktionskatalysator verkürzt werden. Als Basen sind insbesondere tertiäre Amine wie Trimethylamin, Triethylamin, Chinuclidin, 1,4-Diazabicyclo-(2.2.2)-octan, 1,5-Diazabicyclo(4.3.0)non-5-en oder 1,5-Diazabicyclo(5.4.0)undec-7-en geeignet. Als Basen können aber auch anorganische Basen wie Hydride wie Natrium- oder Calciumhydrid, Hydroxide wie Natrium- und Kaliumhydroxid, Carbonate wie Natrium- und Kaliumcarbonat oder Hydrogencarbonate wie Kalium- und Natriumhydrogencarbonat verwendet werden.

Die Endprodukte der Formel I können durch Einengen und/oder Verdampfen des Lösungsmittels isoliert und durch Umkristallisieren oder Zerreiben des festen Rückstandes in Lösungsmitteln, in denen sie sich nicht gut lösen, wie Ether, aromatischen Kohlenwasserstoffen oder chlorierten Kohlenwasserstoffen, gereinigt werden.

Die Zwischenprodukte der Formel XII, III und IV sind bekannt oder können analog zu bekannten Verfahren hergestellt werden. Verfahren zur Herstellung von N-Pyrimidinyl- und N-Triazinylcarbamaten sind beispielsweise in EP-A-0101670 beschrieben. N-Pyrimidinyl- und N-Triazinylisocyanate können aus den entsprechenden 2-Aminopyrimidinen bzw. -triazinen der Formel XI hergestellt werden. Derartige Umsetzungen sowie die Herstellung der Verbindungen der Formel XII sind in EP-A 0 044 808 beschrieben. Verbindungen der Formel XI sind aus EP-A-0 070 804 bekannt. Die Zwischenprodukte der Formel II sind mit Ausnahine derjenigen Verbindung, in welcher R₁, R₂ und R₃ Wasserstoff bedeuten, neu und wurden speziell für die Synthese der Verbindungen der Formel I entwickelt. Sie bilden daher einen Bestandteil der vorliegenden Erfindung.

Die neuen Zwischenprodukte der Formel II können nach verschiedenen, an sich bekannten Verfahren hergestellt werden. So erhält man die Verbindungen der Formel II, indem man ein 3-Halogenpyridin-2-yl-sulfonamid der Formel V 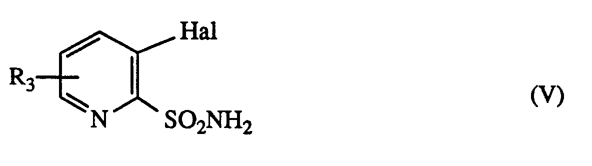
worin R₃ die unter Formel I angegebene Bedeutung hat und Hal für Fluor oder Chlor steht, in Gegenwart einer Base mit einem Amin der Formel VI 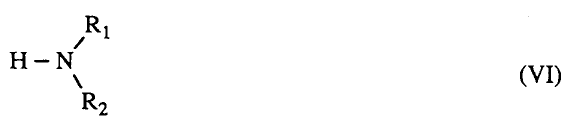
worin R₁ und R₂ die unter Formel I angegebene Bedeutung haben, umsetzt. Für diese Umsetzungen sind diejenigen Verbindungen der Formel V, in denen Hal für Fluor steht, bevorzugt. Derartige Synthesen sind beispielsweise in EP-A- 103 543 beschrieben.

Die Verbindungen der Formel II können auch hergestellt werden, indem man ein 3-Amino-pyridin-2-ylsulfonamid der Formel VII 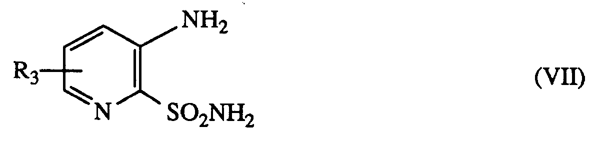
worin R₃ die unter Formel I angegebene Bedeutung hat, in Gegenwart einer Base mit einer Verbindung der Formel VIII

Z-R₁ (VIII)

worin R₁ die unter Formel I angegebene Bedeutung hat und Z für Brom, Jod, CH₃SO₂O-, 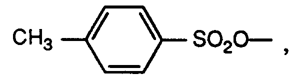
R₁OSO₂O- oder R₂OSO₂O- steht, zur Verbindung der Formel IX 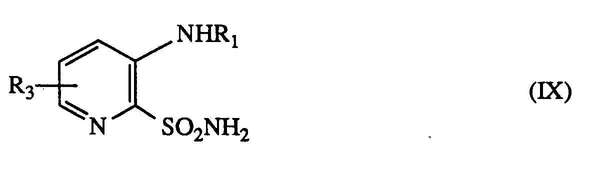
worin R₃ die unter Formel I angegebene Bedeutung hat, umsetzt und diese anschließend mit einer Verbindung der Formel X

Z-R₂ (X)

worin R₂ die unter Formel I angegebene Bedeutung hat und Z die unter Formel VIII angegebene Bedeutung hat, in Gegenwart einer Base in die Verbindung der Formel II überführt. Derartige Umsetzungen sind beispielsweise in Farmaco Ed. scient. **12**, 392 (1957) beschrieben. Die Sulfonamid-Zwischenprodukte der Formeln V und VII sind bekannt. Ihre Herstellung kann beispielsweise analog J.Pharm. Belg. **39**, 217-224 (1984) erfolgen.

Die Wirkstoffe der Formel I werden in der Regel mit Aufwandmengen von 0,001 bis 2 kg/ha insbesondere 0,005 bis 1 kg/ha erfolgreich eingesetzt. Die für die erwünschte Wirkung erforderliche Dosierung kann durch Versuche ermittelt werden. Sie ist abhängig von der Art der Wirkung, dem Entwicklungsstadium der Kulturpflanze und des Unkrauts sowie von der Applikation (Ort, Zeit, Verfahren) und kann, bedingt durch diese Parameter, innerhalb weiter Bereiche variieren.

Bei geringeren Aufwandmengen zeichnen sich die Verbindungen der Formel I durch wuchshemmende und herbizide Eigenschaften aus, die sie ausgezeichnet zum Einsatz in Kulturen von Nutzpflanzen, insbesondere in Getreide, Baumwolle, Soja, Raps, Mais und Reis befähigen, wobei der Einsatz in Maiskultur ganz besonders bevorzugt ist.

Die Erfindung betrifft auch herbizide und pflanzenwachstumsregulierende Mittel, welche einen neuen Wirkstoff der Formel I enthalten, sowie Verfahren zur Hemmung des Pflanzenwuchses.

Pflanzenwachstumsregulatoren sind Substanzen, die in/an der Pflanze agronomisch erwünschte biochemische und/oder physiologische und/oder morphologische Veränderungen bewirken.

Die in den erfindungsgemäßen Mitteln enthaltenen Wirkstoffe beeinflussen das Pflanzenwachstum je nach Applikationszeitpunkt, Dosierung, Applikationsart und Umweltbedingungen in verschiedener Weise. Pflanzenwuchsregulatoren der Formel I können zum Beispiel das vegetative Wachstum von Pflanzen hemmen. Diese Art der Wirkung ist von Interesse auf Rasenflächen, im Zierpflanzenbau, in Obstplantagen, an Straßenböschungen, auf Sport- und Industrieanlagen, aber auch bei der gezielten Hemmung von Nebentrieben wie bei Tabak. Im Ackerbau führt die Hemmung des vegetativen Wachstums bei Getreide über eine Halmverstärkung zu reduziertem Lager, ähnliche agronomische Wirkungen erreicht man in Raps, Sonnenblumen, Mais und anderen Kulturpflanzen. Des weiteren kann durch Hemmung des vegetativen Wachstums die Anzahl Pflanzen pro Fläche erhöht werden. Ein weiteres Einsatzgebiet von Wuchshemmern ist die selektive Kontrolle von bodendeckenden Pflanzen in Plantagen oder weitreihigen Kulturen durch starke Wuchshemmung, ohne diese Bodendecker abzutöten, sodaß die Konkurrenz gegenüber der Hauptkultur ausgeschaltet ist, die agronomisch positiven Effekte wie Erosionsverhinderung, Stickstoffbindung und Bodenlockerung aber erhalten bleiben.

Unter einem Verfahren zur Hemmung des Pflanzenwachstums ist eine Steuerung der natürlichen Pflanzenentwicklung zu verstehen, ohne den von genetischen Eigenschaften determinierten Lebenszyklus der Pflanze im Sinne einer Mutation zu verändern. Das Verfahren der Wuchsregulierung wird zu einem im Einzelfall zu bestimmenden Entwicklungszeitpunkt der Pflanze angewendet. Die Applikation der Wirkstoffe der Formel I kann vor oder nach dem Auflaufen der Pflanzen erfolgen, beispielsweise bereits auf die Samen oder die Sämlinge, auf Wurzeln, Knollen, Stengel, Blätter, Blüten oder andere Pflanzenteile. Dies kann z.B. durch Aufbringen des Wirkstoffes selbst oder in Form eines Mittels auf die Pflanzen und/oder durch Behandlung des Nährmediums der Pflanze (Erdboden) geschehen.

Für die Verwendung der Verbindung der Formel I oder sie enthaltender Mittel zur Regulierung des Pflanzenwachstums kommen verschiedene Methoden und Techniken in Betracht, wie beispielsweise die folgenden:

### i) Samenbeizung

a) Beizung der Samen mit einem als Spritzpulver formulierten Wirkstoff durch Schütteln in einem Gefäß bis zur gleichmäßigen Verteilung auf der Samenoberfläche (Trockenbeizung). Man verwendet dabei bis zu 4 g Wirkstoff der Formel I (bei einer 50 %igen Formulierung: bis zu 8,0 g Spritzpulver) pro 1 kg Saatgut.
b) Beizung der Samen mit einem Emulsionskonzentrat des Wirkstoffs oder mit einer wäßrigen Lösung des als Spritzpulver formulierten Wirkstoffs der Formel I nach Methode a) (Naßbeizung).
c) Beizung durch Tauchen des Saatguts in einer Brühe mit bis zu 1000 ppm Wirkstoff der Formel I während 1 bis 72 Stunden und gegebenenfalls nachfolgendes Trocknen der Samen (Tauchbeizung, Seed soaking).

Die Beizung des Saatguts oder die Behandlung des angekeimten Sämlings sind naturgemäß die bevorzugten Methoden der Applikation, weil die Wirkstoffbehandlung vollständig auf die Zielkultur gerichtet ist. Man verwendet in der Regel 0,001 g bis 4,0 g Aktivsubstanz pro 1 kg Saatgut, wobei man je nach Methodik, die auch den Zusatz anderer Wirkstoffe oder Mikronährstoffe ermöglicht, von den angegebenen Grenzkonzentrationen nach oben oder unten abweichen kann (Wiederholungsbeize).

### ii) Kontrollierte Wirkstoffabgabe

Der Wirkstoff wird in Lösung auf mineralische Granulatträger oder polymerisierte Granulate (Harnstoff/Formaldehyd) aufgezogen und trocknen gelassen. Gegebenenfalls kann ein Überzug aufgebracht werden (Umhüllungsgranulate), der es erlaubt, den Wirkstoff über einen bestimmten Zeitraum dosiert abzugeben.

Die Verbindungen der Formel I werden in unveränderter Form, wie aus der Synthese erhältlich, oder vorzugsweise mit den in der Formulierungstechnik üblichen Hilfsmitteln eingesetzt und werden daher z.B. zu emulgierbaren Konzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet Die Anwendungsverfahren wie Versprühen, Vernebeln, Verstäuben, Benetzen, Verstreuen oder Gießen werden gleich wie die Art der Mittel den angestrebten Zielen und den gegebenen Verhältnissen entsprechend gewählt.

Die Formulierungen, d.h. die den Wirkstoff der Formel I und gegebenenfalls einen oder mehrere feste oder flüssige Zusatzstoffe enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen und gegebenenfalls oberflächenaktiven Verbindungen (Tensiden).

Als Lösungsmittel können in Frage kommen: Aromatische Kohlenwasserstoffe, insbesondere die Fraktionen C₈ bis C₁₂, wie Mischungen von Alkylbenzolen, z.B. Xylolgemische oder alkylierte Naphthaline; aliphatische und cycloaliphatische Kohlenwasserstoffe wie Paraffine, Cyclohexan oder Tetrahydronaphthalin; Alkohole, wie Ethanol, Propanol oder Butanol; Glykole sowie deren Ether und Ester, wie Propylenglykol oder Dipropylenglykolether, Ketone wie Cyclohexanon, Isophoron oder Diacetonalkohol, starke polare Lösungsmittel wie N-Methyl-2-pyrrolidon, Dimethylsulfoxid oder Wasser; Pflanzenöle sowie deren Ester, wie Raps-, Ricinus- oder Sojaöl; gegebenenfalls auch Silikonöle.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinerte Pflanzenruckstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen seien die Alkali-, Erdalkali oder gegebenenfalls substituierten Ammoniumsalze von höheren Fettsäuren (C₁₀-C₂₂), wie z.B. die Na- oder K-Salze der Öl- oder Stearinsäure, oder von natürlichen Fettsäuregemischen genannt, die z.B. aus Kokosnuß- oder Talgöl gewonnen werden können. Ferner sind auch die Fettsäure-methyl-taurinsalze zu erwähnen.

Häufiger werden jedoch sog. synthetische Tenside verwendet, insbesondere Fettalkoholsulfonate, Fettalkoholsulfate, sulfonierte Benzimidazolderivate oder Alkylarylsulfonate.

Die Fettalkoholsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschließt, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxid-Adduktes oder Phospholipide in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Ethylenglykolethergruppen und 10 bis 100 Propylenglykolethergruppen, enthaltenden Polyethylenoxidaddukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nicht ionischer Tenside seien Nonylphenolpolyethoxyethanole, Ricinusölpolyglykolether, Polypropylen-Polyethylenoxidaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi-(2-chlorethyl)-ethylammoniumbromid.

Die in der Formulierungstechnik gebräuchlichen Tenside sind u.a. in folgenden Publikationen beschrieben:
- "Mc Cutcheon's Detergents and Emulsifiers Annual", Mc Publishing Corp., Glen Rock, New Jersey, 1988.
- M. and J. Ash, "Encyclopedia of Surfactants", Vol. I-III, Chemical Publishing Co., New York, 1980-1981.
- Dr. Helmut Stache "Tensid-Taschenbuch", Carl Hanser Verlag, München/Wien 1981.

Die pestiziden Zubereitungen enthalten in der Regel 0,1 bis 99 %, insbesondere 0,1 bis 95 %, Wirkstoff der Formel I, 1 bis 99 % eines festen oder flüssigen Zusatzstoffes und 0 bis 25 %, insbesondere 0,1 bis 25 % eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren z.B. gegebenenfalls epoxydierte Pflanzenöle (epoxydiertes Kokosnußöl, Rapsöl oder Sojaöl), Entschäumer, z.B. Silikonöl, Konservierungsmittel, Viskositätsregulatoren, Bindemittel, Haftmittel sowie Dünger oder andere Wirkstoffe zur Erzielung spezieller Effekte enthalten.

Insbesondere setzen sich bevorzugte Formulierungen folgendermaßen zusammen: (% = Gewichtsprozent)

| Emulgierbare Konzentrate: | |
|---|---|
| Aktiver Wirkstoff: | 1 bis 90 %, vorzugsweise 5 bis 20% |
| oberflächenaktives Mittel: | 1 bis 30%, vorzugsweise 10 bis 20 % |
| flüssiges Trägermittel: | 50 bis 94 %, vorzugsweise 70 bis 85 % |

| Stäube: | |
|---|---|
| Aktiver Wirkstoff: | 0,1 bis 10 %, vorzugsweise 0,1 bis 1 % |
| festes Trägermittel: | 99,9 bis 90 %, vorzugsweise 99,9 bis 99 % |

| Suspensions-Konzentrate: | |
|---|---|
| Aktiver Wirkstoff: | 5 bis 75 %, vorzugsweise 10 bis 50 % |
| Wasser: | 94 bis 24 %, vorzugsweise 88 bis 30 % |
| oberflächenaktives Mittel: | 1 bis 40 %, vorzugsweise 2 bis 30 % |

| Benetzbare Pulver: | |
|---|---|
| Aktiver Wirkstoff: | 0,5 bis 90 %, vorzugsweise 1 bis 80 % |
| oberflächenaktives Mittel: | 0,5 bis 20 %, vorzugsweise 1 bis 15 % |
| festes Trägermaterial: | 5 bis 95 %, vorzugsweise 15 bis 90 % |

| Granulate: | |
|---|---|
| Aktiver Wirkstoff: | 0,5 bis 30 %, vorzugsweise 3 bis 15 % |
| festes Trägermittel: | 99,5 bis 70 %, vorzugsweise 97 bis 85 % |

### Herstellungsbeispiele:

### Beispiel H1: Herstellung von

### N-(3-n-Butylamino-pyridin-2-yl-sulfonyl)-N'-(4,6-dimethoxy- pyrimidin-2-yl)-harnstoff (Verbindung Nr. 1.001):

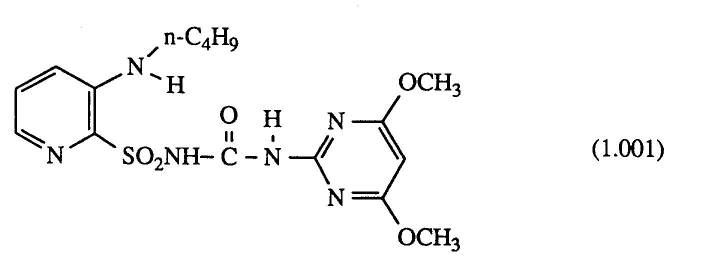

Zu einer Mischung von 2,75 g 3-n-Butylamino-pyridin-2-yl-sulfonamid in 40 ml Acetonitril werden nacheinander zugegeben: 3,5 g N-(4,6-Dimethoxy-pyrimidin-2-yl)-phenylcarbamat und 1,97 ml 1,5-Diazabicyclo[5.4.0]undec-5-en. Die Reaktionsmischung wird 30 Minuten gerührt, eingedampft, der ölige Rückstand mit 10 ml 2N Salzsäure und nachfolgend mit 10 ml Eis-Wasser und wenig Diethylether verrieben und filtriert. Der Filterrückstand wird anschließend mit wenig Wasser und Diethylether gewaschen und getrocknet.
Man erhält 4,39 g N-(3-n-Butylamino-pyridin-2-yl-sulfonyl)-N'-(4,6-dimethoxypyrimidin-2-yl)-harnstoff (Verbindung Nr. 1.001) mit einem Schmelzpunkt von 118-119°C.

### Beispiel H2: Herstellung von 3-n-Butylamino-pyridin-2-yl-sulfonamid (Zwischenprodukt Nr. 2.001):

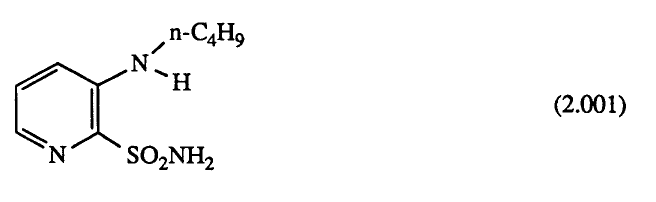

Zu einer Mischung von 8,81 g 3-Fluor-pyridin-2-yl-sulfonamid in 10 ml Tetrahydrofuran (THF) werden 49,6 ml n-Butylamin gegeben. Nach 18-stündigem Rühren der Reaktionsmischung bei einer Temperatur von 60°C wird die Reaktionsmischung eingedampft, der ölige Rückstand mit 100 ml Eis-Wasser verrieben, filtriert, zuerst mit wenig kaltem Wasser und anschließend mit n-Hexan gewaschen. Nach Trocknen bei einer Temperatur von 45°C im Vakuum erhält man 8,8 g 3-n-Butylamino-pyridin-2-yl-sulfonamid (Zwischenprodukt Nr. 2.001) mit einem Schmelzpunkt von 100-101°C.

### Beispiel H3: 3-Allylamino-pyridin-2-yl-sulfonamid (Zwischenprodukt Nr. 2.002):

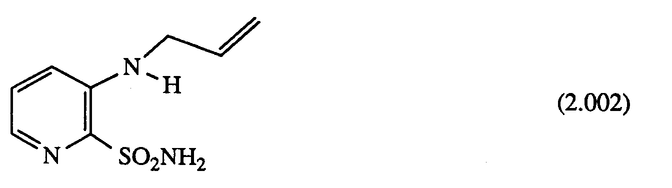

5,28 g 3-Fluor-pyridin-2-yl-sulfonamid und 18,03 ml Allylamin werden in einem Druckgefäß 6 Stunden bei einer Temperatur von 125°C gerührt. Anschließend wird die Reaktionslösung eingeengt, das erhaltene Kristallisat in Methylenchlorid und wenig Acetonitril gelöst und mit Methylenchlorid/Essigester (4:1) als Eluiergemisch und Silicagel als Trägermaterial chromatographisch gereinigt. Man erhält 5,1 g 3-Allylamino-pyridin-2-yl-sulfonamid (Zwischenprodukt Nr. 2.002) mit einem Schmelzpunkt von 134-135°C.

In analoger Weise werden die in den angeschlossenen Tabellen aufgelisteten Verbindungen der Formel I sowie die Zwischenprodukte der Formel II hergestellt.

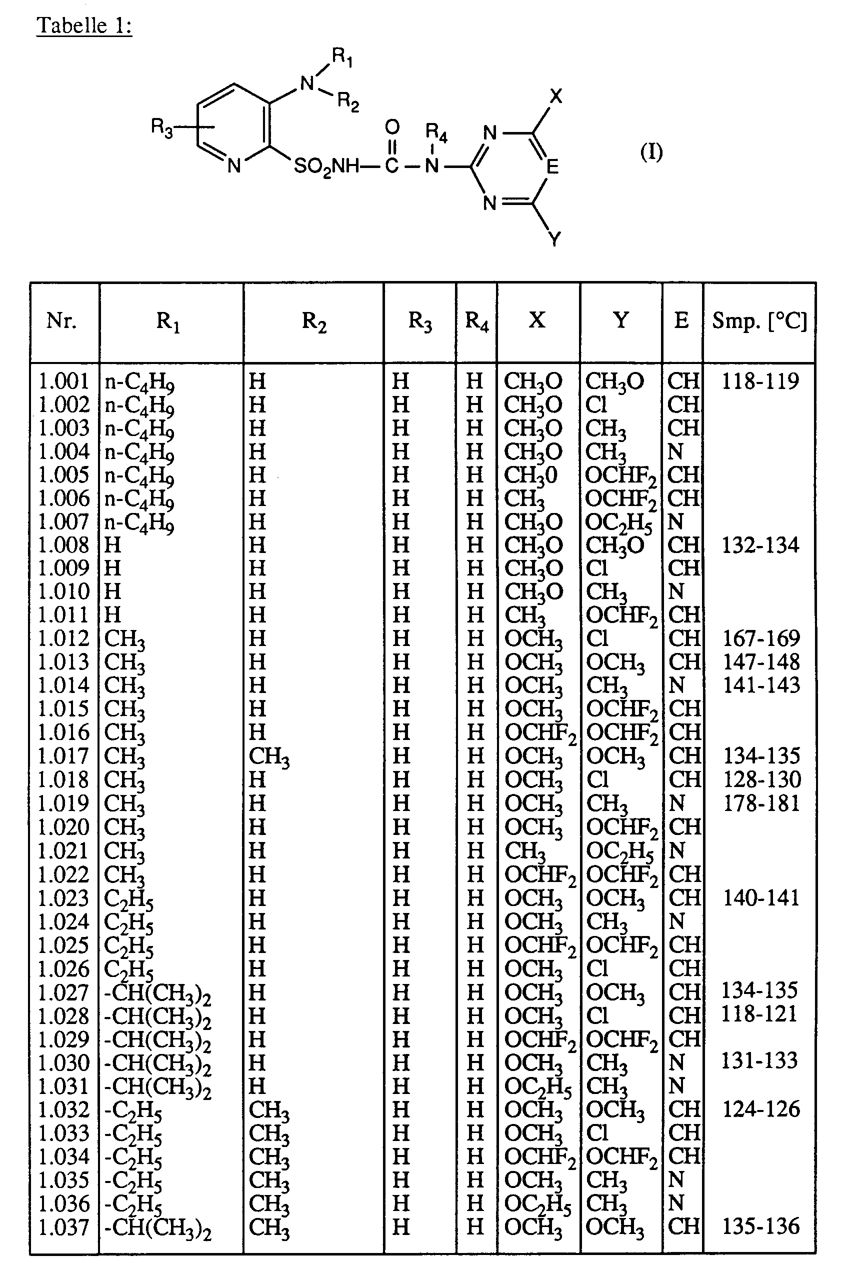

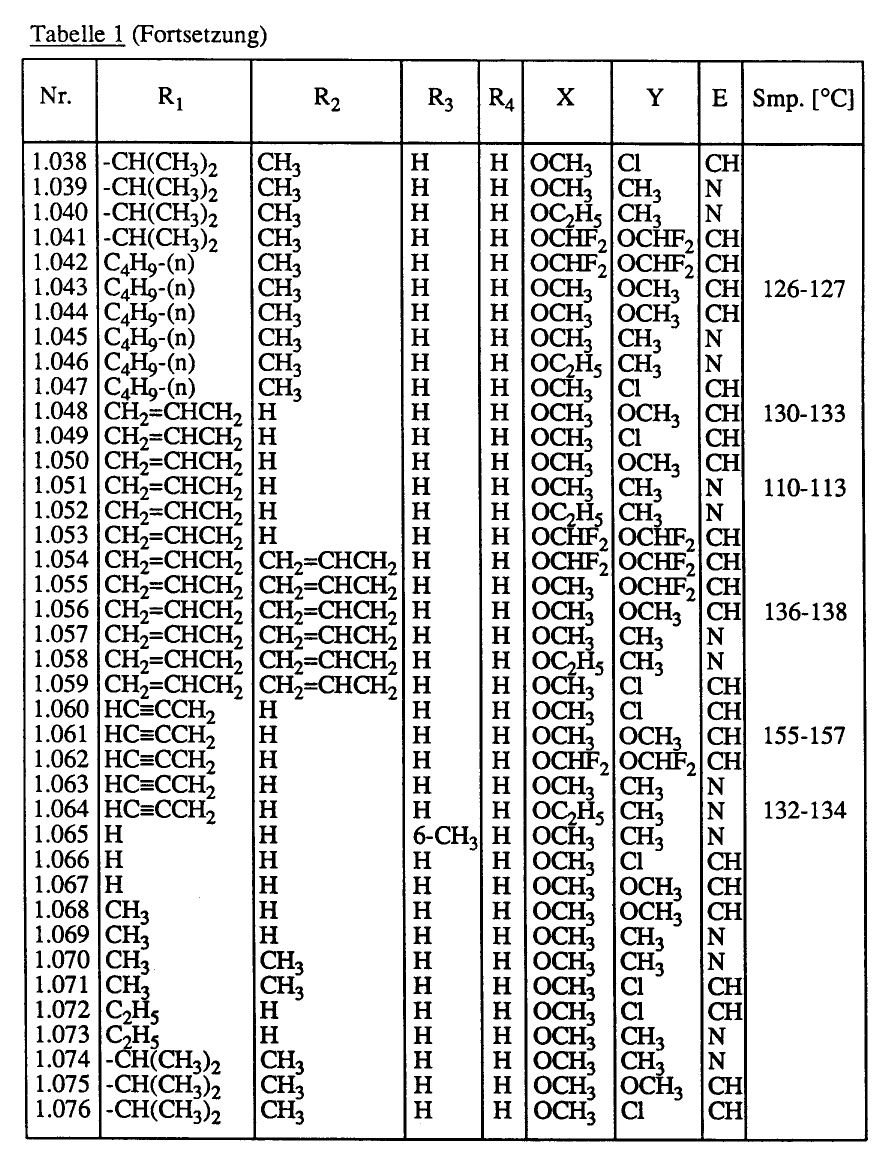

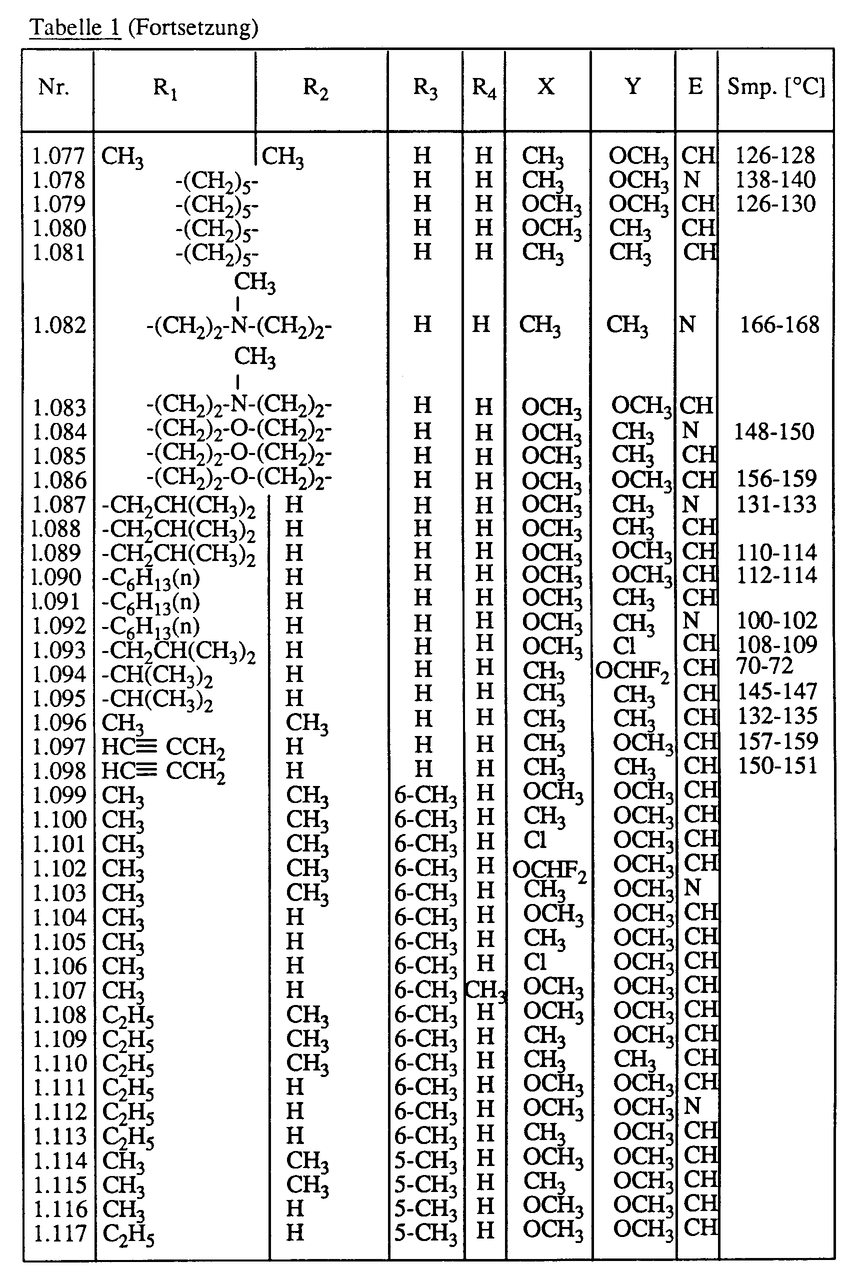

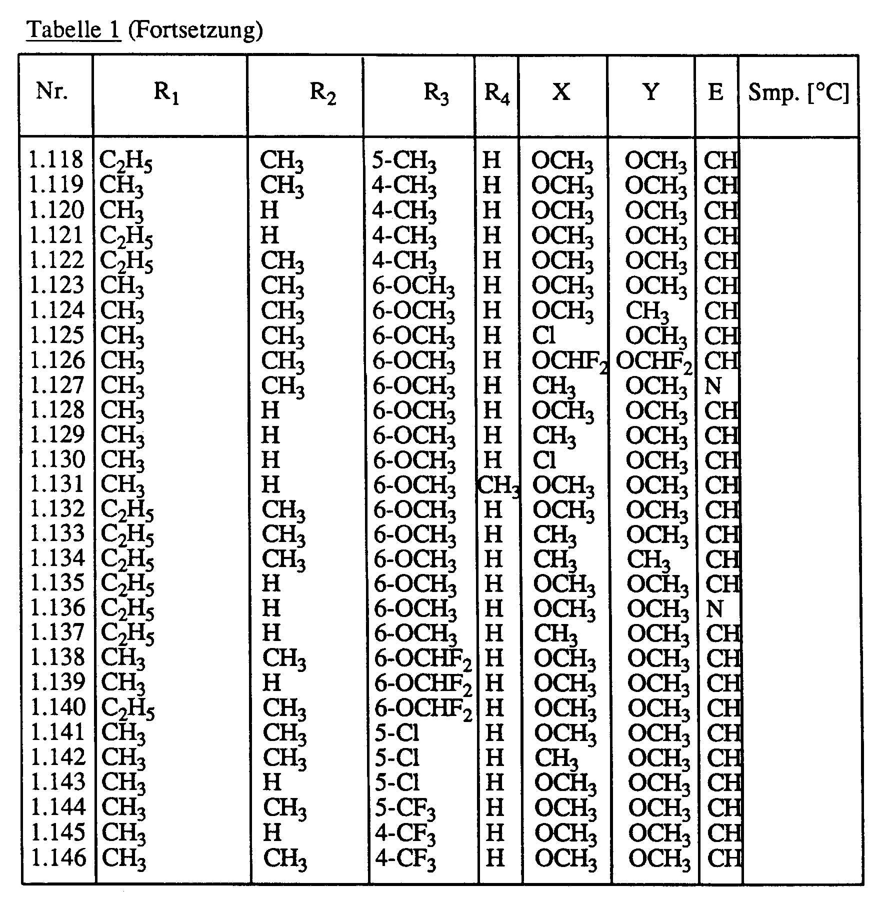

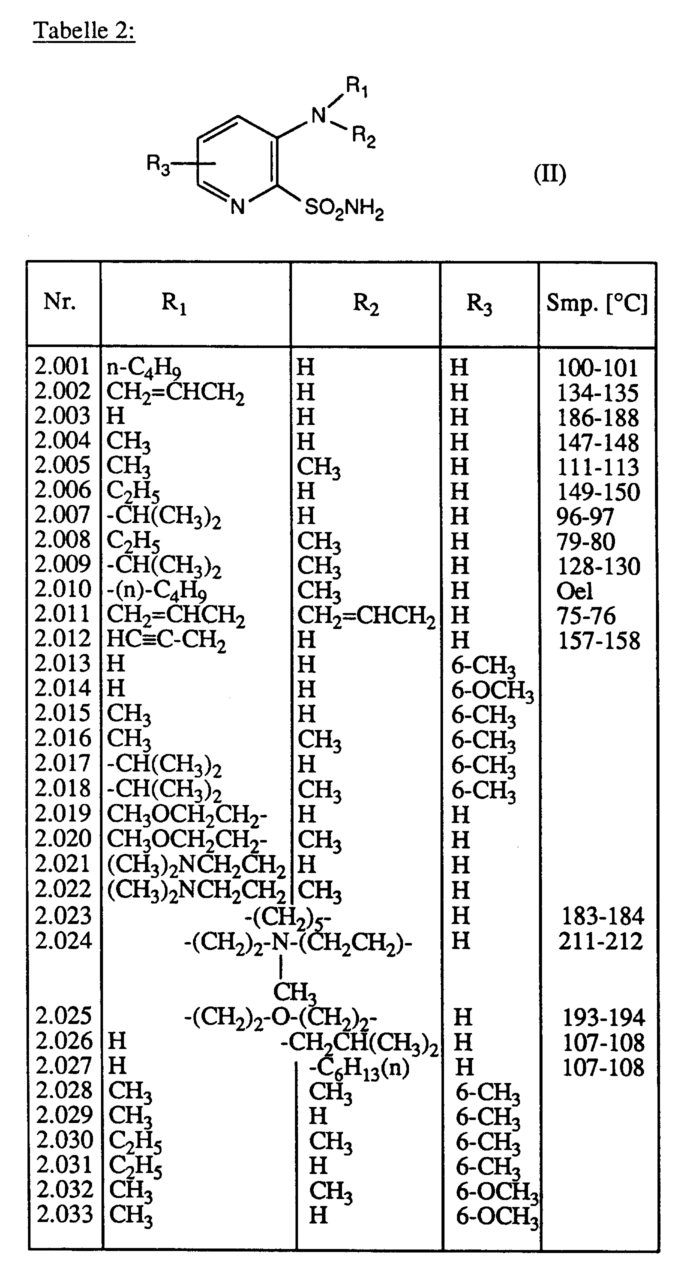

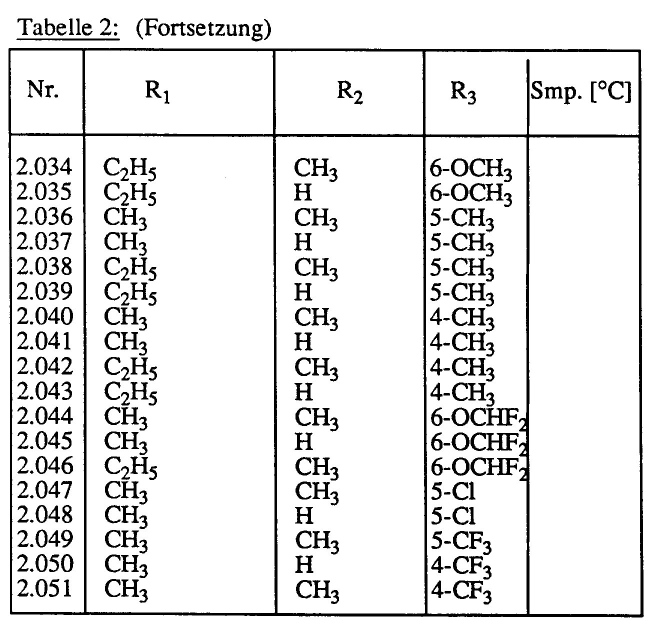

### Biologische Beispiele

Beispiel B1: Herbizidwirkung vor dem Auflaufen der Pflanzen
Kunststofftöpfe werden mit expandiertem Vermiculit (Dichte: 0,135 g/cm³, Wasseradsorptionsvermögen: 0,565 l/l) gefüllt. Nach dem Sättigen des nicht adsorptiven Vermiculits mit einer wäßrigen Wirkstoffemulsion in deionisiertem Wasser, die die Wirkstoffe in einer Konzentration von 70,8 ppm enthält, werden Samen der folgenden Pflanzen auf die Oberfläche gesät: Nasturtium officinalis, Agrostis tenuis, Stellaria media und Digitaria sanguinalis. Die Versuchsgefäße werden anschließend in einer Klimakammer bei einer Temperatur von 20 °C, einer Beleuchtung von ca. 20 kLux und einer relativen Luftfeuchtigkeit von 70 % gehalten. Während einer Keimphase von 4 bis 5 Tagen werden die Töpfe zur Erhöhung der örtlichen Luftfeuchtigkeit mit lichtdurchlässigem Material abgedeckt und mit deionisiertem Wasser gegossen. Nach dem 5. Tag wird dem Gießwasser 0,5 % eines handelsüblichen Flüssigdüngers zugesetzt. 12 Tage nach der Aussaat wird der Versuch ausgewertet und die Wirkung auf die Versuchspflanzen nach dem folgenden Maßstab bewertet:
- 1: : Pflanze nicht gekeimt oder total abgestorben
- 2-3: : sehr starke Wirkung
- 4-6: : mittlere Wirkung
- 7-8: : schwache Wirkung
- 9: : keine Wirkung (wie unbehandelte Kontrolle)

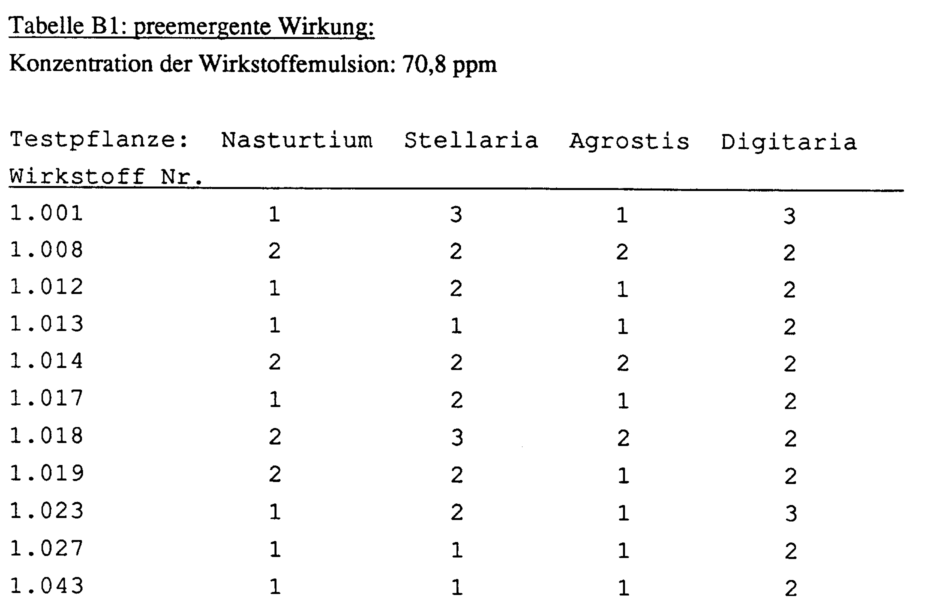

Beispiel B2: Post-emergente Herbizid-Wirkung (Kontaktherbizid)
Eine Anzahl Unkräuter, sowohl monokotyle wie dikotyle, wurden nach dem Auflaufen (im 4- bis 6-Blattstadium) mit einer wäßrigen Wirkstoffdispersion in einer Dosierung von 8-500 g Wirksubstanz pro Hektar auf die Pflanzen gespritzt und diese bei 24°-26°C und 45-60 % relativer Luftfeuchtigkeit gehalten.

Nach 3 Wochen wird die Herbizidwirkung mit einem neunstufigen (1 = vollständige Schädigung, 9 = keine Wirkung) Boniturschema im Vergleich in einer unbehandelten Kontrollgruppe bewertet. Boniturnoten von 1 bis 4 (insbesondere 1 bis 3) weisen auf eine gute bis sehr gute Herbizidwirkung hin. Boniturnoten von 6 bis 9 (insbesondere von 7 bis 9) weisen auf eine gute Toleranz (insbesondere bei Kulturpflanzen) hin.

In diesem Versuch zeigen die Verbindungen der Formel I starke Herbizidwirkung.

Beispiel B3: Herbizidwirkung für Wasserreis (paddy) Die Wasserunkräuter Echinochloa crus galli und Monocharia vag. werden in Plastikbechern (60 cm² Oberfläche, 500 ml Volumen) ausgesät. Nach der Saat wird bis zur Erdoberfläche mit Wasser aufgefüllt. 3 Tage nach der Saat wird der Wasserspiegel bis leicht über die Erdoberfläche erhöht (3-5 mm). Die Applikation erfolgt 3 Tage nach der Saat durch eine Spritzung der Prüfsubstanzen auf die Gefäße. Die verwendete Dosis entspricht einer Wirkstoffmenge von 500 g AS pro Hektar. Die Pflanzenbecher werden dann im Gewächshaus unter optimalen Wachstumsbedingungen für die Reisunkräuter aufgestellt, d.h. bei 25°-30°C und hoher Luftfeuchtigkeit.

Die Auswertung der Versuche findet 3 Wochen nach Applikation statt. Die Verbindungen der Formel I schädigen dabei die Unkräuter.

### Formulierungsbeispiele für Wirkstoffe der Formel I

### (% = Gewichtsprozent)

| 1. Spritzpulver | a) | b) | c) |
|---|---|---|---|
| Wirkstoff gemäß Tabelle 1 | 20 % | 50 % | 0,5 % |
| Na-Ligninsulfonat | 5 % | 5 % | 5 |
| Na-Laurylsulfat | 3 % | - | - % |
| Na-Diisobutylnaphthalinsulfonat | - | 6 % | 6 % |
| Octylphenolpolyethylenglykolether (7-8 Mol AeO) | - | 2 % | 2 |
| Hochdisperse Kieselsäure | 5 % | 27 % | 27 % |
| Kaolin | 67 % | - % | - |
| Natriumchlorid | - | - | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 2. Emulsions-Konzentrate | a) | b) |
|---|---|---|
| Wirkstoff gemäß Tabelle 1 | 10% | 1 % |
| Ca-Dodecylbenzolsulfonat | 3 % | 3 % |
| Octylphenolpolyethylenglykolether (4-5 Mol AeO) | 3 % | 3 % |
| Ricinusöl-polyethylenglykolether (36 Mol AeO) | 4 % | 4 |
| Cyclohexanon | 30 | 10 % |
| Xylolgemisch | 50 % | 79 % |

Aus solchen Konzentraten können durch Verdünnen mit Wasser Emulsionen jeder gewünschten Konzentration hergestellt werden.

| 3. Stäubemittel | a) | b) |
|---|---|---|
| Wirkstoff gemäß Tabelle 1 | 0,1 % | 1 % |
| Talkum | 99,9% | - |
| Kaolin | - | 99 % |

Durch inniges Vermischen der Trägerstoffe mit dem Wirkstoff erhält man gebrauchsfertige Stäubemittel.

| 4. Extruder-Granulat | a) | b) |
|---|---|---|
| Wirkstoff gemäß Tabelle 1 | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschließend im Luftstrom getrocknet.

| 5. Umhüllungs-Granulat | |
|---|---|
| Wirkstoff gemäß Tabelle 1 | 3 % |
| Polyäthylenglykol (MG200) | 3 % |
| Kaolin | 94 % |

Der fein gemahlene Wirkstoff wird in einem Mischer auf das mit Polyethylenglykol angefeuchtete Kaolin gleichmäßig aufgetragen. Auf diese Weise erhält man staubfreie Umhüllungs-Granulate.

| 6. Suspensions-Konzentrat | a) | b) |
|---|---|---|
| Wirkstoff gemäß Tabelle 1 | 5 % | 40 % |
| Ethylenglykol | 10 % | 10 % |
| Nonylphenolpolyethylenglykolether (15 Mol AeO) | 1 % | 6 % |
| Na-Ligninsulfonat | 5 % | 10 % |
| Carboxymethylcellulose | 1 % | 1 % |
| 37 %ige wäßrige Formaldehyd-Lösung | 0,2 % | 0,2 % |
| Silikonöl in Form einer 75%-igen wäßrigen Emulsion | 0,8 % | 0,8 % |
| Wasser | 77 % | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

| 7. Salzlösung | |
|---|---|
| Wirkstoff gemäß Tabelle 1 | 5 % |
| Isopropylamin | 1 % |
| Octylphenolpolyethylenglycolether (78 Mol AeO) | 3 % |
| Wasser | 91% |

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen, in bekannter Weise verarbeitet. Die Anwendungsverfahren wie Versprühen, Vernebeln, Verstäuben, Verstreuen oder Gießen werden gleich wie die Art der Mittel den angestrebten Zielen und den gegebenen Verhältnissen entsprechend gewählt.

## Patentansprüche (Patentansprüche für folgende(n) Vertragsstaat(en): CH, DE, FR, GB, IT, LI)

1. N-Pyridinsulfonyl-N'-pyrimidinyl- und -triazinylharnstoffe der Formel I 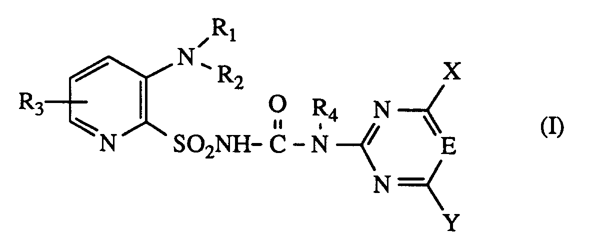 worin
R₁ und R₂ unabhängig voneinander Wasserstoff, C₁-C₈-Alkyl oder C₃-C₈-Cycloalkyl; durch Halogen ein- oder mehrfach substituiertes C₂-C₈-Alkyl oder C₃-C₈-Cycloalkyl; durch -NR₅R₆, C₁-C₄-Alkoxy oder C₁-C₄-Alkyl-S(O)ₙ- substituiertes C₁-C₈-Alkyl oder C₃-C₈-Cycloalkyl; durch C₂-C₄-Alkenyl, C₅-C₆-Cycloalkenyl, C₂-C₆-Alkinyl oder C₄-C₈-Alkadienyl substituiertes C₁-C₄-Alkyl oder C₃-C₆-Cycloalkyl; wobei die C₂-C₄-Alkenyl-, C₅-C₆-Cycloalkenyl- und C₄-C₈-Alkadienylreste durch Halogen ein- oder mehrfach substituiert sein können; oder R₁ und R₂ zusammen eine 4-5-gliedrige C₄-C₁₀-Alkylenkette, die durch Sauerstoff, Schwefel oder N-R₇ unterbrochen sein kann;
R₃ Wasserstoff, Halogen, C₁-C₄-Alkyl, C₁-C₄-Alkoxy oder C₁-C₄-Alkylthio; durch Halogen ein- oder mehrfach substituiertes C₁-C₄-Alkyl, C₁-C₄-AIkoxy oder C₁-C₄-Alkylthio;
R₄, R₅, R₆ und R₇ unabhängig voneinander Wasserstoff oder C₁-C₄-Alkyl;
n 0, 1 oder 2;
X C₁-C₃-Alkyl, durch Halogen ein- bis dreifach substituiertes C₁-C₃-Alkyl; C₁-C₃-Alkoxy oder durch Halogen ein- bis dreifach substituiertes C₁-C₃-Alkoxy;
Y Halogen, C₁-C₃-Alkyl, durch Halogen ein- bis dreifach substituiertes C₁-C₃-Alkyl; C₁-C₃-Alkoxy, durch Halogen ein- bis dreifach substituiertes C₁-C₃-Alkoxy; Cyclopropyl, Methylamino oder Dimethylamino; und
E Stickstoff oder die Methingruppe bedeuten,
sowie die Salze dieser Verbindungen.

2. Verbindungen der Formel I gemaß Anspruch 1, dadurch gekennzeichnet, daß
R₁ und R₂ unabhängig voneinander Wasserstoff, C₁-C₈-Alkyl oder C₃-C₈-Cycloalkyl; durch Halogen ein- oder mehrfach substituiertes C₂-C₈-Alkyl oder C₃-C₈-Cycloalkyl; durch -NR₅R₆, C₁-C₄-Alkoxy oder C₁-C₄-Alkyl-S(O)ₙ- substituiertes C₁-C₈-Alkyl oder C₃-C₈-Cycloalkyl; durch C₂-C₄-Alkenyl, C₅-C₆-Cycloalkenyl, C₂-C₆-Alkinyl oder C₄-C₈-Alkadienyl substituiertes C₁-C₄-Alkyl oder C₃-C₆-Cycloalkyl; wobei die C₂-C₄-Alkenyl-, C₅-C₆-Cycloalkenyl- und C₄-C₈-Alkadienylreste durch Halogen ein- oder mehrfach substituiert sein können; oder R₁ und R₂ zusammen eine 4-5-gliedrige C₄-C₅-Alkylenkette, die durch Sauerstoff, Schwefel oder N-R₇ unterbrochen sein kann; bedeuten.

3. Verbindungen der Formel I gemäß Anspruch 1, dadurch gekennzeichnet, daß
R₁ und R₂ unabhängig voneinander Wasserstoff, C₁-C₈-Alkyl oder C₃-C₈-Cycloalkyl; durch Halogen ein- oder mehrfach substituiertes C₂-C₈-Alkyl oder C₃-C₈-Cycloalkyl; durch -NR₅R₆, C₁-C₄-Alkoxy oder C₁-C₄-Alkyl-S(O)ₙ- substituiertes C₁-C₈-Alkyl oder C₃-C₈-Cycloalkyl; durch C₂-C₄-Alkenyl, C₅-C₆-Cycloalkenyl, C₂-C₆-Alkinyl oder C₄-C₈-Alkadienyl substituiertes C₁-C₄-Alkyl oder C₃-C₆-Cycloalkyl; wobei die C₂-C₄-Alkenyl-, C₅-C₆-Cycloalkenyl- und C₄-C₈-Alkadienylreste durch Halogen ein- oder mehrfach substituiert sein können; bedeuten.

4. Verbindungen der Formel I gemäß Anspruch 1, dadurch gekennzeichnet daß mindestens einer der Substituenten R₁, R₂ und R₃ verschieden von Wasserstoff ist.

5. Verbindungen der Formel I gemäß Anspruch 1, dadurch gekennzeichnet, daß
R₁ und R₂ unabhängig voneinander Wasserstoff, C₁-C₆-Alkyl oder C₃-C₆-Cycloalkyl; durch Halogen ein- bis dreifach substituiertes C₂-C₆-Alkyl oder C₃-C₆-Cycloalkyl; durch -NR₅R₆, C₁-C₄-Alkoxy oder C₁-C₄-Alkyl-S(O)ₙ- substituiertes C₁-C₆-Alkyl oder C₃-C₆-Cycloalkyl; durch C₂-C₄-Alkenyl, C₅-C₆-Cycloalkenyl, C₂-C₄-Alkinyl oder C₄-C₈-Alkadienyl substituiertes C₁-C₄-Alkyl oder C₃-C₆-Cycloalkyl; wobei die C₂-C₄-Alkenyl-, C₅-C₆-Cycloalkenyl- und C₄-C₈-Alkadienylreste durch Halogen ein- bis dreifach substituiert sein können; und
R₃ Wasserstoff, Halogen, C₁-C₄-Alkyl, C₁-C₄-Alkoxy oder C₁-C₄-Alkylthio; oder durch Halogen ein- bis dreifach substituiertes C₁-C₄-Alkyl, C₁-C₄-Alkoxy oder C₁-C₄-Alkylthio bedeutet.

6. Verbindungen der Formel I gemäß Anspruch 1, dadurch gekennzeichnet, daß X für C₁-C₃-Alkyl, C₁-C₃-Alkoxy oder durch Halogen ein- bis dreifach substituiertes C₁-C₃-Alkoxy; und
Y für Chlor, C₁-C₃-Alkyl, C₁-C₃-Alkoxy oder durch Halogen ein- bis dreifach substituiertes C₁-C₃-Alkoxy steht.

7. Verbindungen der Formel I gemäß Anspruch 1, dadurch gekennzeichnet, daß
X für Methyl, Methoxy, Ethoxy oder Difluormethoxy; und
Y für Methyl, Methoxy, Ethoxy, Difluormethoxy oder Chlor steht.

8. Verbindungen der Formel I gemäß Anspruch 1, dadurch gekennzeichnet, daß X für Methoxy oder Ethoxy; und Y für Methyl oder Methoxy steht.

9. Verbindungen der Formel I gemäß Anspruch 1, dadurch gekennzeichnet, daß E die Methinbrücke bedeutet.

10. Verbindungen der Formel I gemäß Anspruch 1, dadurch gekennzeichnet, daß R₃ in der 6-Stellung des Pyridinringes steht.

11. Verbindungen der Formel I gemäß Anspruch 1, dadurch gekennzeichnet, daß R₃ Wasserstoff bedeutet.

12. Verbindungen der Formel I gemäß Anspruch 1, dadurch gekennzeichnet, daß
R₂ und R₃ für Wasserstoff und R₁ für C₁-C₆-Alkyl oder C₃-C₆-Cycloalkyl; durch Halogen ein- bis dreifach substituiertes C₂-C₆-Alkyl oder C₃-C₆-Cycloalkyl; durch -NR₅R₆, C₁-C₄-Alkoxy oder C₁-C₄-Alkyl-S(O)ₙ- substituiertes C₁-C₆-Alkyl oder C₃-C₆-Cycloalkyl; durch C₂-C₄-Alkenyl, C₅-C₆-Cycloalkenyl, C₂-C₄-Alkinyl oder C₄-C₈-Alkadienyl substituiertes C₁-C₄-Alkyl oder C₃-C₆-Cycloalkyl; wobei die C₂-C₄-Alkenyl-, C₅-C₆-Cycloalkenyl- und C₄-C₈-Alkadienylreste durch Halogen ein- bis dreifach substituiert sein können; steht.

13. Verbindungen der Formel I gemäß Anspruch 1, dadurch gekennzeichnet, daß R₁ C₁-C₆-Alkyl, Allyl oder Propargyl und R₂ Wasserstoff, Methyl oder Allyl bedeutet.

14. N-(3-n-Butylamino-pyridin-2-yl-sulfonyl)-N'-(4,6-dimethoxy-pyrimidin-2-yl)-harnstoff gemäß Anspruch 1.

15. Verfahren zur Herstellung der Verbindungen der Formel I gemäß Anspruch 1, dadurch gekennzeichnet, daß man
a) ein Pyridylsulfonamid der Formel II 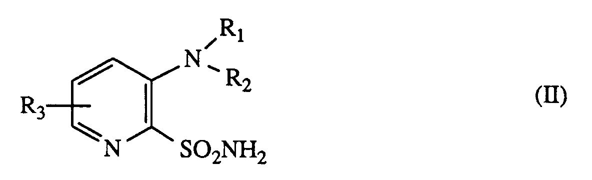 worin R₁, R₂ und R₃ die unter Formel I im Anspruch 1 angegebene Bedeutung haben, mit einem N-Pyrimidinyl- oder N-Triazinylcarbamat der Formel III 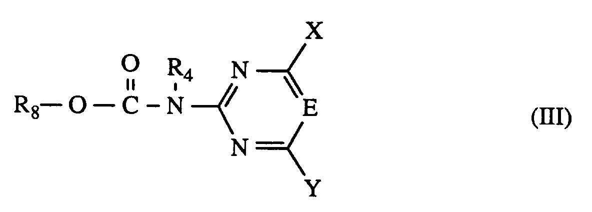 worin X, Y, R₄ und E die unter Formel I in Anspruch 1 angegebene Bedeutung haben und R₈ C₁-C₄-Alkyl, oder Phenyl, welches durch C₁-C₄-Alkyl oder Halogen substituiert sein kann, bedeutet; in Gegenwart einer Base umsetzt, oder
b) ein Pyridylsulfonamid der Formel XII 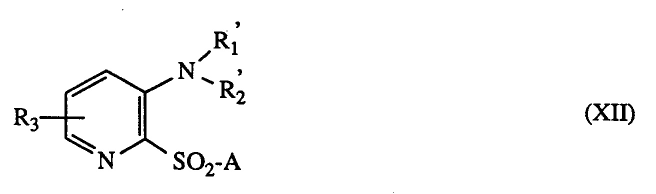 worin R₁', R₂' die unter R₁ und R₂ in Formel I im Anspruch 1 angegebene Bedeutung mit Ausnahme von Wasserstoff haben, R₃ die unter Formel I im Anspruch 1 angegebene Bedeutung hat und A 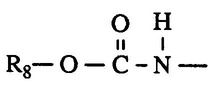 oder O=C=N- bedeutet, wobei R₈ die unter Formel III angegebene Bedeutung hat, in Gegenwart einer Base mit einem 2-Aminopyrimidin- oder -triazin der Formel XI 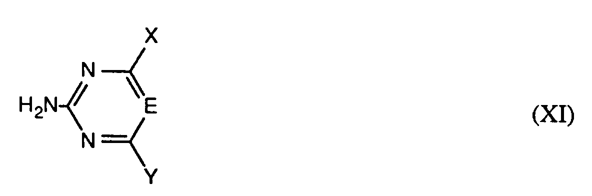 worin E, X und Y die unter Formel I angegebene Bedeutung haben, umsetzt.

16. Verfahren zur Herstellung der Verbindungen der Formel Ia, 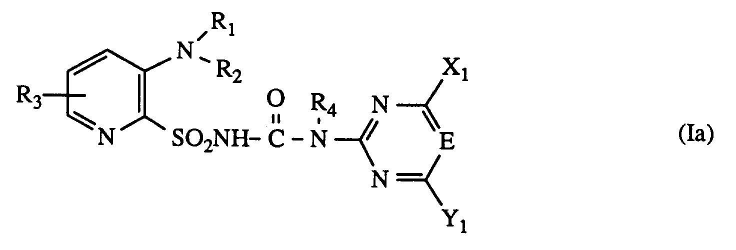 worin X₁ und Y₁ unabhängig voneinander für Chlor oder Difluormethoxy stehen und R₁, R₂, R₃, R₄ und E die unter Formel I in Anspruch 1 angegebene Bedeutung haben, dadurch gekennzeichnet, daß man ein Pyridylsulfonamid der Formel II 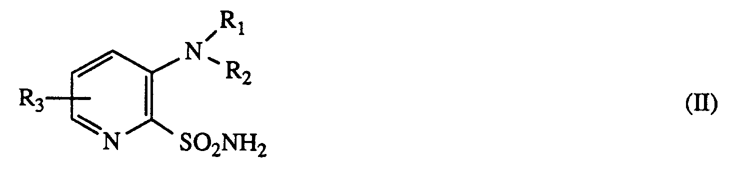 worin R₁, R₂ und R₃ die unter Formel I im Anspruch 1 angegebene Bedeutung haben, in Gegenwart einer Base mit einem Pyrimidinyl- oder Triazinylisocyanat der Formel IV 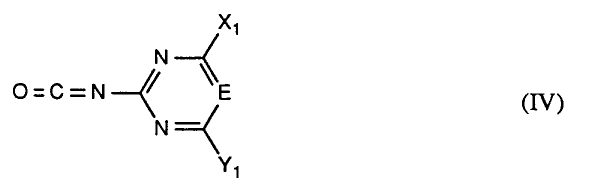 worin X₁ und Y₁ die unter Formel Ia angegebene Bedeutung haben und E die unter Formel I angegebene Bedeutung hat, umsetzt.

17. Verbindungen der Formel II 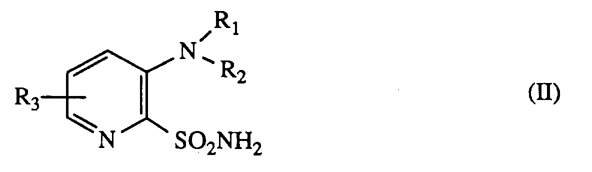 worin R₁, R₂ und R₃ die unter Formel I im Anspruch 1 angegebene Bedeutung haben, mit der Maßgabe, daß R₁, R₂ und R₃ nicht gleichzeitig Wasserstoff bedeuten.

18. Ein herbizides und den Pflanzenwuchs hemmendes Mittel, gekennzeichnet durch ein Gehalt an einem oder mehreren Sulfonylharnstoffen der Formel I, gemäß Anspruch 1.

19. Mittel gemäß Anspruch 16, dadurch gekennzeichnet, daß es zwischen 0,1 % und 95 % Wirkstoff der Formel I gemäß Anspruch 1 enthält.

20. Verfahren zur Bekämpfung unerwünschten Pflanzenwachstums, dadurch gekennzeichnet, daß man einen Wirkstoff der Formel I, gemäß Anspruch 1, oder ein diesen Wirkstoff enthaltendes Mittel in einer wirksamen Menge auf die Pflanzen oder deren Lebensraum appliziert.

21. Verfahren gemäß Anspruch 20, dadurch gekennzeichnet, daß man eine Wirkstoffmenge zwischen 0,001 und 2 kg pro Hektar appliziert.

22. Verfahren zur Hemmung des Pflanzenwachstums, dadurch gekennzeichnet, daß man einen Wirkstoff der Formel I, gemäß Anspruch 1, oder ein diesen Wirkstoff enthaltendes Mittel in einer wirksamen Menge auf die Pflanzen oder deren Lebensraum appliziert.

23. Verfahren gemäß Anspruch 20, zur selektiven pre- oder post-emergenten Bekämpfung von Unkräutern in Nutzpflanzenkulturen.

## Patentansprüche (Patentansprüche für folgende(n) Vertragsstaat(en): ES)

1. Verfahren zur Herstellung von N-Pyridinsulfonyl-N'-pyrimidinyl und -triazinylharnstoffen der Formel I, 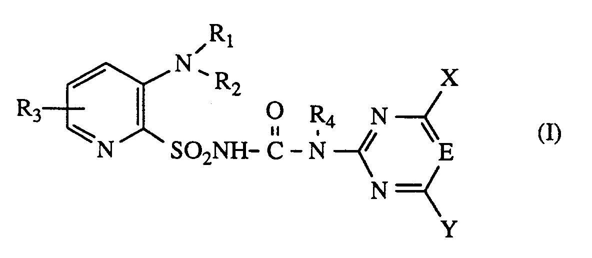 worin
R₁ und R₂ unabhängig voneinander Wasserstoff, C₁-C₈-Alkyl oder C₃-C₈-Cycloalkyl; durch Halogen ein- oder mehrfach substituiertes C₂-C₈-Alkyl oder C₃-C₈-Cycloalkyl; durch -NR₅R₆, C₁-C₄-Alkoxy oder C₁-C₄-Alkyl-S(O)ₙ- substituiertes C₁-C₈-Alkyl oder C₃-C₈-Cycloalkyl; durch C₂-C₄-Alkenyl, C₅-C₆-Cycloalkenyl, C₂-C₆-Alkinyl oder C₄-C₈-Alkadienyl substituiertes C₁-C₄-Alkyl oder C₃-C₆-Cycloalkyl; wobei die C₂-C₄-Alkenyl-, C₅-C₆-Cycloalkenyl- und C₄-C₈-Alkadienylreste durch Halogen ein- oder mehrfach substituiert sein können; oder R₁ und R₂ zusammen eine 4-5-gliedrige C₄-C₁₀-Alkylenkette, die durch Sauerstoff, Schwefel oder N-R₇ unterbrochen sein kann;
R₃ Wasserstoff, Halogen, C₁-C₄-Alkyl, C₁-C₄-Alkoxy oder C₁-C₄-Alkylthio; durch Halogen ein- oder mehrfach substituiertes C₁-C₄-Alkyl, C₁-C₄-Alkoxy oder C₁-C₄-Alkylthio;
R₄, R₅, R₆ und R₇ unabhängig voneinander Wasserstoff oder C₁-C₄-Alkyl;
n 0, 1 oder 2;
X C₁-C₃-Alkyl, durch Halogen ein- bis dreifach substituiertes C₁-C₃-Alkyl;
C₁-C₃-Alkoxy oder durch Halogen ein- bis dreifach substituiertes C₁-C₃-Alkoxy;
Y Halogen, C₁-C₃-Alkyl, durch Halogen ein- bis dreifach substituiertes C₁-C₃-Alkyl;
C₁-C₃-Alkoxy, durch Halogen ein- bis dreifach substituiertes C₁-C₃-Alkoxy; Cyclopropyl, Methylamino oder Dimethylamino; und
E Stickstoff oder die Methingruppe bedeuten,
sowie die Salze dieser Verbindungen, dadurch gekennzeichnet, daß man
a) ein Pyridylsulfonamid der Formel II 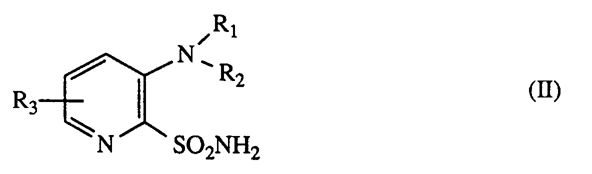 worin R₁, R₂ und R₃ die unter Formel I angegebene Bedeutung haben, mit einem N-Pyrimidinyl- oder N-Triazinylcarbamat der Formel III 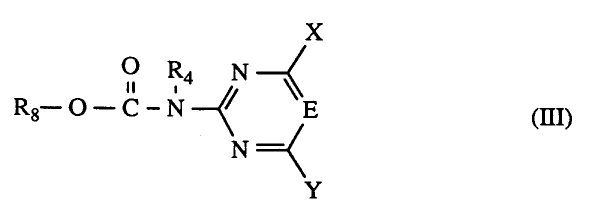 worin X, Y, R₄ und E die unter Formel I angegebene Bedeutung haben und R₈ C₁-C₄-Alkyl, oder Phenyl, welches durch C₁-C₄-Alkyl oder Halogen substituiert sein kann, bedeutet; in Gegenwart einer Base umsetzt, oder
b) ein Pyridylsulfonamid der Formel XII 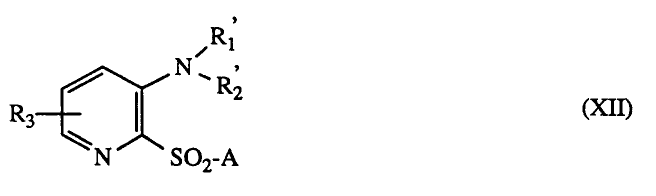 worin R₁', R₂' die oben unter R₁ und R₂ angegebene Bedeutung mit Ausnahme von Wasserstoff haben, R₃ die unter Formel I angegebene Bedeutung hat und A 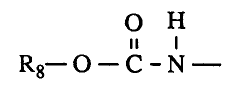 oder O=C=N- bedeutet, wobei R₈ die unter Formel III angegebene Bedeutung hat, in Gegenwart einer Base mit einem 2-Aminopyrimidin- oder -triazin der Formel XI 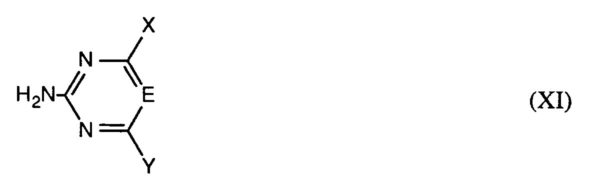 worin E, X und Y die unter Formel I angegebene Bedeutung haben, umsetzt.

2. Verfahren nach Anspruch 1, dadurch gekennzeichnet, daß in den Verbindungen der Formel I
R₁ und R₂ unabhängig voneinander Wasserstoff, C₁-C₈-Alkyl oder C₃-C₈-Cycloalkyl; durch Halogen ein- oder mehrfach substituiertes C₂-C₈-Alkyl oder C₃-C₈-Cycloalkyl; durch -NR₅R₆, C₁-C₄-Alkoxy oder C₁-C₄-Alkyl-S(O)ₙ- substituiertes C₁-C₈-Alkyl oder C₃-C₈-Cycloalkyl; durch C₂-C₄-Alkenyl, C₅-C₆-Cycloalkenyl, C₂-C₆-Alkinyl oder C₄-C₈-Alkadienyl substituiertes C₁-C₄-Alkyl oder C₃-C₆-Cycloalkyl; wobei die C₂-C₄-Alkenyl-, C₅-C₆-Cycloalkenyl- und C₄-C₈-Alkadienylreste durch Halogen ein- oder mehrfach substituiert sein können; oder R₁ und R₂ zusammen eine 4-5-gliedrige C₄-C₅-Alkylenkette, die durch Sauerstoff, Schwefel oder N-R₇ unterbrochen sein kann; bedeuten.

3. Verfahren nach Anspruch 1, dadurch gekennzeichnet, daß in den Verbindungen der Formel I
R₁ und R₂ unabhängig voneinander Wasserstoff, C₁-C₈-Alkyl oder C₃-C₈-Cycloalkyl; durch Halogen ein- oder mehrfach substituiertes C₂-C₈-Alkyl oder C₃-C₈-Cycloalkyl; durch -NR₅R₆, C₁-C₄-Alkoxy oder C₁-C₄-Alkyl-S(O)ₙ- substituiertes C₁-C₈-Alkyl oder C₃-C₈-Cycloalkyl; durch C₂-C₄-Alkenyl, C₅-C₆-Cycloalkenyl, C₂-C₆-Alkinyl oder C₄-C₈-Alkadienyl substituiertes C₁-C₄-Alkyl oder C₃-C₆-Cycloalkyl; wobei die C₂-C₄-Alkenyl-, C₅-C₆-Cycloalkenyl- und C₄-C₈-Alkadienylreste durch Halogen ein- oder mehrfach substituiert sein können; bedeuten.

4. Verfahren nach Anspruch 1, dadurch gekennzeichnet, daß in den Verbindungen der Formel I mindestens einer der Substituenten R₁, R₂ und R₃ verschieden von Wasserstoff ist.

5. Verfahren nach Anspruch 1, dadurch gekennzeichnet, daß in den Verbindungen der Formel I
R₁ und R₂ unabhängig voneinander Wasserstoff, C₁-C₆-Alkyl oder C₃-C₆-Cycloalkyl; durch Halogen ein- bis dreifach substituiertes C₂-C₆-Alkyl oder C₃-C₆-Cycloalkyl; durch -NR₅R₆, C₁-C₄-Alkoxy oder C₁-C₄-Alkyl-S(O)ₙ- substituiertes C₁-C₆-Alkyl oder C₃-C₆-Cycloalkyl; durch C₂-C₄-Alkenyl, C₅-C₆-Cycloalkenyl, C₂-C₄-Alkinyl oder C₄-C₈-Alkadienyl substituiertes C₁-C₄-Alkyl oder C₃-C₆-Cycloalkyl; wobei die C₂-C₄-Alkenyl-, C₅-C₆-Cycloalkenyl- und C₄-C₈-Alkadienylreste durch Halogen ein- bis dreifach substituiert sein können; und
R₃ Wasserstoff, Halogen, C₁-C₄-Alkyl, C₁-C₄-Alkoxy oder C₁-C₄-Alkylthio; oder durch Halogen ein- bis dreifach substituiertes C₁-C₄-Alkyl, C₁-C₄-Alkoxy oder C₁-C₄-Alkylthio bedeutet.

6. Verfahren nach Anspruch 1, dadurch gekennzeichnet, daß in den Verbindungen der Formel I
X für C₁-C₃-Alkyl, C₁-C₃-Alkoxy oder durch Halogen ein- bis dreifach substituiertes C₁-C₃-Alkoxy; und
Y für Chlor, C₁-C₃-Alkyl, C₁-C₃-Alkoxy oder durch Halogen ein- bis dreifach substituiertes C₁-C₃-Alkoxy steht.

7. Verfahren nach Anspruch 1, dadurch gekennzeichnet, daß in den Verbindungen der Formel I
X für Methyl, Methoxy, Ethoxy oder Difluormethoxy; und
Y für Methyl, Methoxy, Ethoxy, Difluormethoxy oder Chlor steht.

8. Verfahren nach Anspruch 1, dadurch gekennzeichnet, daß in den Verbindungen der Formel I X für Methoxy oder Ethoxy; und Y für Methyl oder Methoxy steht.

9. Verfahren nach Anspruch 1, dadurch gekennzeichnet, daß in den Verbindungen der Formel I E die Methinbrücke bedeutet.

10. Verfahren nach Anspruch 1, dadurch gekennzeichnet, daß in den Verbindungen der Formel I R₃ in der 6-Stellung des Pyridinringes steht.

11. Verfahren nach Anspruch 1, dadurch gekennzeichnet, daß in den Verbindungen der Formel I R₃ Wasserstoff bedeutet.

12. Verfahren nach Anspruch 1, dadurch gekennzeichnet, daß in den Verbindungen der Formel I
R₂ und R₃ für Wasserstoff und R₁ für C₁-C₆-Alkyl oder C₃-C₆-Cycloalkyl; durch Halogen ein- bis dreifach substituiertes C₂-C₆-Alkyl oder C₃-C₆-Cycloalkyl; durch -NR₅R₆, C₁-C₄-Alkoxy oder C₁-C₄-Alkyl-S(O)ₙ- substituiertes C₁-C₆-Alkyl oder C₃-C₆-Cycloalkyl; durch C₂-C₄-Alkenyl, C₅-C₆-Cycloalkenyl, C₂-C₄-Alkinyl oder C₄-C₈-Alkadienyl substituiertes C₁-C₄-Alkyl oder C₃-C₆-Cycloalkyl; wobei die C₂-C₄-Alkenyl-, C₅-C₆-Cycloalkenyl- und C₄-C₈-Alkadienylreste durch Halogen ein- bis dreifach substituiert sein können; steht.

13. Verfahren nach Anspruch 1, dadurch gekennzeichnet, daß in den Verbindungen der Formel I
R₁ C₁-C₆-Alkyl, Allyl oder Propargyl und R₂ Wasserstoff, Methyl oder Allyl bedeutet.

14. Verfahren nach Anspruch 1, dadurch gekennzeichnet, daß die Verbindung der Formel I N-(3-n-Butylamino-pyridin-2-yl-sulfonyl)-N'-(4,6-dimethoxy-pyrimidin-2-yl)-harnstoff bedeutet.

15. Verfahren zur Herstellung der Verbindungen der Formel Ia, 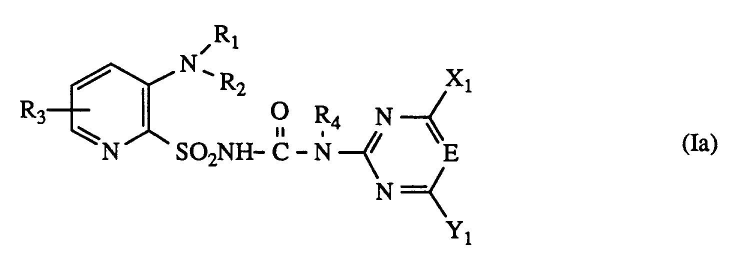 worin X₁ und Y₁ unabhängig voneinander für Chlor oder Difluormethoxy stehen und R₁, R₂, R₃, R₄ und E die unter Formel I in Anspruch 1 angegebene Bedeutung haben, dadurch gekennzeichnet, daß man ein Pyridylsulfonamid der Formel II 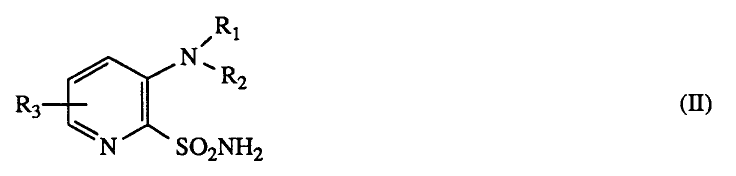 worin R₁, R₂ und R₃ die unter Formel I im Anspruch 1 angegebene Bedeutung haben, in Gegenwart einer Base mit einem Pyrimidinyl- oder Triazinylisocyanat der Formel IV 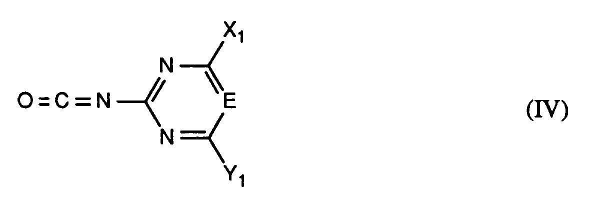 worin X₁und Y₁ die unter Formel Ia angegebene Bedeutung haben und E die unter Formel I angegebene Bedeutung hat, umsetzt.

16. Verfahren zur Bekämpfung unerwünschten Pflanzenwachstums, dadurch gekennzeichnet, daß man einen Wirkstoff der Formel I, gemäß Anspruch 1, oder ein diesen Wirkstoff enthaltendes Mittel in einer wirksamen Menge auf die Pflanzen oder deren Lebensraum appliziert.

17. Verfahren gemäß Anspruch 16, dadurch gekennzeichnet, daß man eine Wirkstoffmenge zwischen 0,001 und 2 kg pro Hektar appliziert.

18. Verfahren zur Hemmung des Pflanzenwachstums, dadurch gekennzeichnet, daß man einen Wirkstoff der Formel I, gemäß Anspruch 1, oder ein diesen Wirkstoff enthaltendes Mittel in einer wirksamen Menge auf die Pflanzen oder deren Lebensraum appliziert.

19. Verfahren gemäß Anspruch 16, zur selektiven pre- oder post-emergenten Bekämpfung von Unkräutern in Nutzpflanzenkulturen.

## Claims (Claims for the following Contracting State(s): CH, DE, FR, GB, IT, LI)

1. An N-pyridinesulfonyl-N'-pyrimidinyl- or -triazinylurea of the formula I 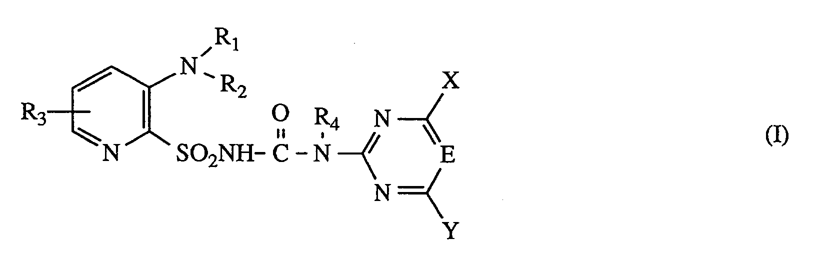 in which R₁ and R₂ independently of one another are hydrogen, C₁-C₈alkyl or C₃-C₈cycloalkyl; C₂-C₈alkyl or C₃-C₈cycloalkyl, each of which is monosubstituted or polysubstituted by halogen; C₁-C₈alkyl or C₃-C₈cycloalkyl, each of which is substituted by -NR₅R₆, C₁-C₄alkoxy or C₁-C₄alkyl-S(O)ₙ-; C₁-C₄alkyl or C₃-C₆cycloalkyl, each of which is substituted by C₂-C₄alkenyl, C₅-C₆cycloalkenyl, C₂-C₆alkynyl or C₄-C₈alkadienyl; it being possible for the C₂-C₄alkenyl, C₅-C₆cycloalkenyl and C₄-C₈alkadienyl radicals to be monosubstituted or polysubstituted by halogen; or R₁ and R₂ together are a 4-5-membered C₄-C₁₀alkylene chain which can be interrupted by oxygen, sulfur or N-R₇; R₃ is hydrogen, halogen, C₁-C₄alkyl, C₁-C₄alkoxy or C₁-C₄alkylthio; C₁-C₄alkyl, C₁-C₄alkoxy or C₁-C₄alkylthio, each of which is monosubstituted or polysubstituted by halogen; R₄, R₅, R₆ and R₇ independently of one another are hydrogen or C₁-C₄alkyl: n is 0, 1 or 2; X is C₁-C₃alkyl, or C₁-C₃alkyl which is monosubstituted to trisubstituted by halogen; C₁-C₃alkoxy, or C₁-C₃alkoxy which is monosubstituted to trisubstituted by halogen; Y is halogen, C₁-C₃alkyl or C₁-C₃alkyl which is monosubstituted to trisubstituted by halogen; C₁-C₃alkoxy, or C₁-C₃alkoxy which is monosubstituted to trisubstituted by halogen; cyclopropyl, methylamino or dimethylamino; and E is nitrogen or the methine group, and the salts of this.

2. A compound of the formula I according to claim 1, in which R₁ and R₂ independently of one another are hydrogen, C₁-C₈alkyl or C₃-C₈cycloalkyl; C₂-C₈alkyl or C₃-C₈cycloalkyl which are monosubstituted or polysubstituted by halogen; C₁-C₈alkyl or C₃-C₈cycloalkyl which are substituted by -NR₅R₆, C₁-C₄alkoxy or C₁-C₄alkyl-S(O)ₙ-;
C₁-C₄alkyl or C₃-C₆cycloalkyl which are substituted by C₂-C₄alkenyl, C₅-C₆cycloalkenyl, C₂-C₆alkynyl or C₄-C₈alkadienyl; it being possible for the C₂-C₄alkenyl, C₅-C₆cycloalkenyl and C₄-C₈alkadienyl radicals to be monosubstituted or polysubstituted by halogen; or R₁ and R₂ together are a 4-5-membered C₄-C₅alkylene chain which can be interrupted by oxygen, sulfur or N-R₇.

3. A compound of the formula I according to claim 1, in which R₁ and R₂ independently of one another are hydrogen, C₁-C₈alkyl or C₃-C₈cycloalkyl; C₂-C₈alkyl or C₃-C₈cycloalkyl which are monosubstituted or polysubstituted by halogen; C₁-C₈alkyl or C₃-C₈cycloalkyl which are substituted by -NR₅R₆, C₁-C₄alkoxy or C₁-C₄alkyl-S(O)ₙ-;
C₁-C₄alkyl or C₃-C₆cycloalkyl which are substituted by C₂-C₄alkenyl, C₅-C₆cycloalkenyl, C₂-C₆alkynyl or C₄-C₈alkadienyl; it being possible for the C₂-C₄alkenyl, C₅-C₆cycloalkenyl and C₄-C₈alkadienyl radicals to be monosubstituted or polysubstituted by halogen.

4. A compound of the formula I according to claim 1, in which at least one of the substituents R₁, R₂ and R₃ is other than hydrogen.

5. A compound of the formula I according to claim 1, in which R₁ and R₂ independently of one another are hydrogen, C₁-C₆alkyl or C₃-C₆cycloalkyl; C₂-C₆alkyl or C₃-C₆cycloalkyl which are monosubstituted to trisubstituted by halogen; C₁-C₆alkyl or C₃-C₆cycloalkyl which are substituted by -NR₅R₆, C₁-C₄alkoxy or C₁-C₄alkyl-S(O)ₙ-; C₁-C₄alkyl or C₃-C₆cycloalkyl which are substituted by C₂-C₄alkenyl, C₅-C₆cycloalkenyl, C₂-C₄alkynyl or C₄-C₈alkadienyl; it being possible for the C₂-C₄alkenyl, C₅-C₆cycloalkenyl and C₄-C₈alkadienyl radicals to be monosubstituted to trisubstituted by halogen; and R₃ is hydrogen, halogen, C₁-C₄alkyl, C₁-C₄alkoxy or C₁-C₄alkylthio; or C₁-C₄alkyl, C₁-C₄alkoxy or C₁-C₄alkylthio which are monosubstituted to trisubstituted by halogen.

6. A compound of the formula I according to claim 1, in which X is C₁-C₃alkyl, C₁-C₃alkoxy or C₁-C₃alkoxy which is monosubstituted to trisubstituted by halogen; and Y is chlorine, C₁-C₃alkyl, C₁-C₃alkoxy or C₁-C₃alkoxy which is monosubstituted to trisubstituted by halogen.

7. A compound of the formula I according to claim 1, in which X is methyl, methoxy, ethoxy or difluoromethoxy; and Y is methyl, methoxy, ethoxy, difluoromethoxy or chlorine.

8. A compound of the formula I according to claim 1, in which X is methoxy or ethoxy; and Y is methyl or methoxy.

9. A compound of the formula I according to claim 1, in which E is the methine bridge.

10. A compound of the formula I according to claim 1, in which R₃ is in the 6-position of the pyridine ring.

11. A compound of the formula I according to claim 1, in which R₃ is hydrogen.

12. A compound of the formula I according to claim 1, in which R₂ and R₃ are hydrogen and R₁ is C₁-C₆alkyl or C₃-C₆cycloalkyl; C₂-C₆alkyl or C₃-C₆cycloalkyl which are monosubstituted to trisubstituted by halogen; C₁-C₆alkyl or C₃-C₆cycloalkyl which are substituted by -NR₅R₆, C₁-C₄alkoxy or C₁-C₄alkyl-S(O)ₙ-; C₁-C₄alkyl or C₃-C₆cycloalkyl which are substituted by C₂-C₄alkenyl, C₅-C₆cycloalkenyl, C₂-C₄alkynyl or C₄-C₈alkadienyl; it being possible for the C₂-C₄alkenyl, C₅-C₆cycloalkenyl and C₄-C₈alkadienyl radicals to be monosubstituted to trisubstituted by halogen.

13. A compound of the formula I according to claim 1, in which R₁ is C₁-C₆alkyl, allyl or propargyl, and R₂ is hydrogen, methyl or allyl.

14. N-(3-n-Butylaminopyridin-2-ylsulfonyl)-N'-(4,6-dimethoxypyrimidin-2-yl)urea according to claim 1.

15. A process for the preparation of a compound of the formula I according to claim 1, which comprises reacting
a) a pyridylsulfonamide of the formula II 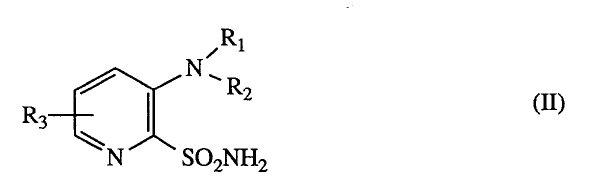 in which R₁, R₂ and R₃ are as defined under formula I in claim 1, with an N-pyrimidinyl-or N-triazinylcarbamate of the formula III 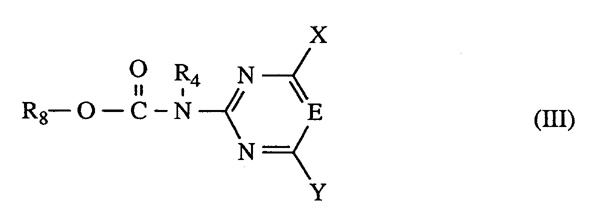 in which X, Y, R₄ and E are as defined under formula I in claim 1 and R₈ is C₁-C₄alkyl, or phenyl which can be substituted by C₁-C₄alkyl or halogen, in the presence of a base, or
b) a pyridylsulfonamide of the formula XII 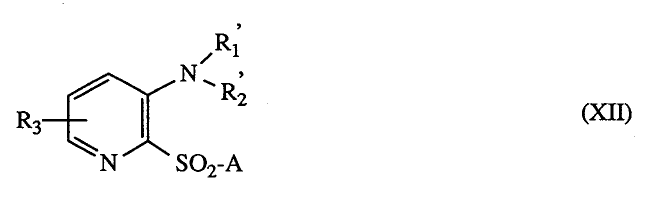 in which R₁', R₂' are as defined under R₁ and R₂ in formula I in claim 1, with the exception of hydrogen, R₃ is as defined under formula I in claim 1, and A is 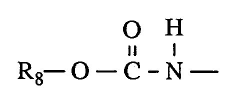 or O=C=N-, R₈ being as defined under formula III, with a 2-aminopyrimidine or -triazine of the formula XI 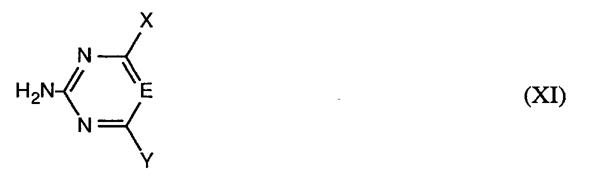 in which E, X and Y are as defined under formula I, in the presence of a base.

16. A process for the preparation of a compound of the formula Ia 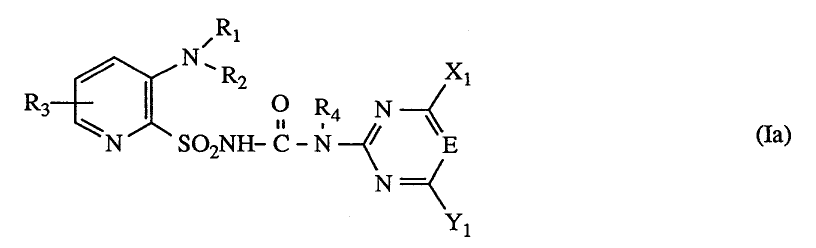 in which X₁ and Y₁ independently of one another are chlorine or difluoromethoxy and R₁, R₂, R₃, R₄ and E are as defined under formula I in claim 1, which comprises reacting a pyridylsulfonamide of the formula II 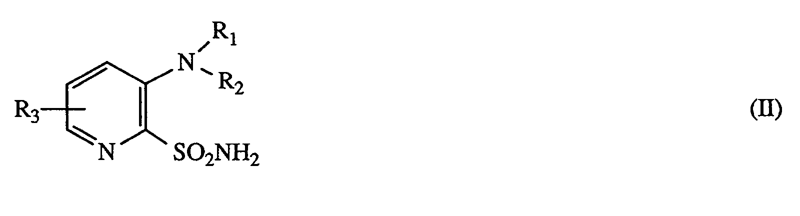 in which R₁, R₂ and R₃ are as defined under formula I in claim 1, with a pyrimidinyl or triazinyl isocyanate of the formula IV 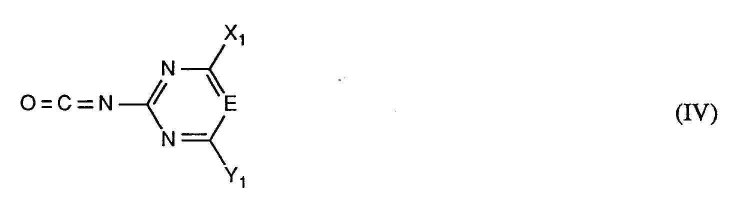 in which X₁ and Y₁ are as defined under formula Ia and E is as defined under formula I, in the presence of a base.

17. A compound of the formula II 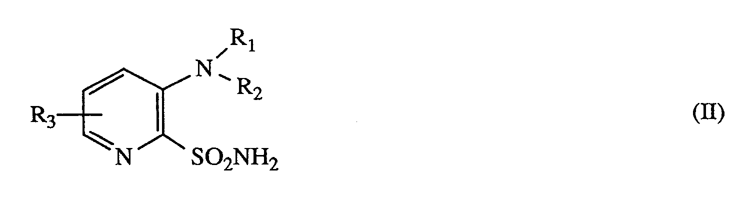 in which R₁, R₂ and R₃ are as defined under formula I in claim 1, with the proviso that R₁, R₂ and R₃ are not simultaneously hydrogen.

18. A herbicidal and plant-growth-inhibiting composition, which comprises one or more sulfonylureas of the formula I according to claim 1.

19. A composition according to claim 16, which comprises between 0.1 % and 95 % of active substance of the formula I according to claim 1.

20. A method of controlling undesired plant growth, which comprises applying an effective amount of an active substance of the formula I according to claim 1, or a composition comprising this active substance, to the plants or their environment.

21. A method according to claim 20, in which an amount of active substance of between 0.001 and 2 kg is applied per hectare.

22. A method of inhibiting plant growth, which comprises applying an effective amount of an active substance of the formula I according to claim 1, or a composition comprising this active substance, to the plants or their environment.

23. A method according to claim 20 for selective pre-emergence or post-emergence control of weeds in crops of useful plants.

## Claims (Claims for the following Contracting State(s): ES)

1. A process for the preparation of an N-pyridinesulfonyl-N'-pyrimidinyl- or -triazinylurea of the formula I 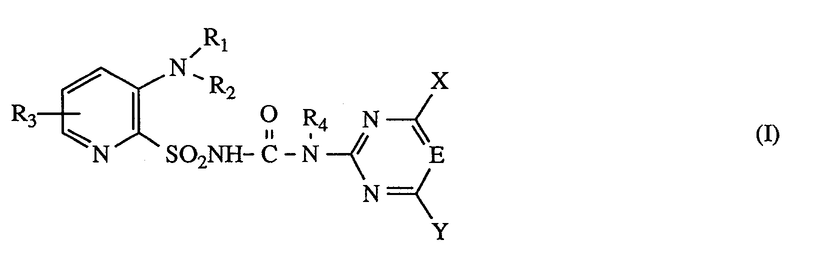 in which R₁ and R₂ independently of one another are hydrogen, C₁-C₈alkyl or C₃-C₈cycloalkyl; C₂-C₈alkyl or C₃-C₈cycloalkyl, each of which is monosubstituted or polysubstituted by halogen; C₁-C₈alkyl or C₃-C₈cycloalkyl, each of which is substituted by -NR₅R₆, C₁-C₄alkoxy or C₁-C₄alkyl-S(O)ₙ-; C₁-C₄alkyl or C₃-C₆cycloalkyl, each of which is substituted by C₂-C₄alkenyl, C₅-C₆cycloalkenyl, C₂-C₆alkynyl or C₄-C₈alkadienyl; it being possible for the C₂-C₄alkenyl, C₅-C₆cycloalkenyl and C₄-C₈alkadienyl radicals to be monosubstituted or polysubstituted by halogen; or R₁ and R₂ together are a 4-5-membered C₄-C₁₀alkylene chain which can be interrupted by oxygen, sulfur or N-R₇; R₃ is hydrogen, halogen, C₁-C₄alkyl, C₁-C₄alkoxy or C₁-C₄alkylthio; C₁-C₄alkyl, C₁-C₄alkoxy or C₁-C₄alkylthio, each of which is monosubstituted or polysubstituted by halogen; R₄, R₅, R₆ and R₇ independently of one another are hydrogen or C₁-C₄alkyl; n is 0, 1 or 2; X is C₁-C₃alkyl, or C₁-C₃alkyl which is monosubstituted to trisubstituted by halogen; C₁-C₃alkoxy, or C₁-C₃alkoxy which is monosubstituted to trisubstituted by halogen; Y is halogen, C₁-C₃alkyl or C₁-C₃alkyl which is monosubstituted to trisubstituted by halogen; C₁-C₃alkoxy, or C₁-C₃alkoxy which is monosubstituted to trisubstituted by halogen; cyclopropyl, methylamino or dimethylamino; and E is nitrogen or the methine group, and the salts of this, which comprises reacting
a) a pyridylsulfonamide of the formula II 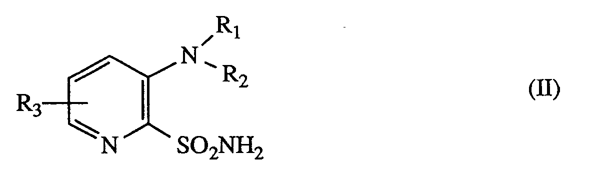 in which R₁, R₂ and R₃ are as defined under formula I, with an N-pyrimidinyl- or N-triazinylcarbamate of the formula III 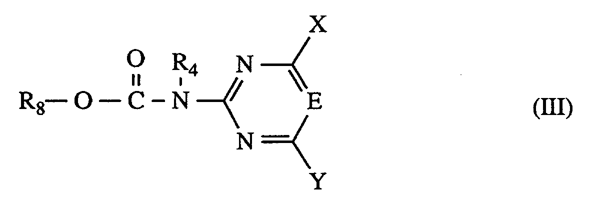 in which X, Y, R₄ and E are as defined under formula I and R₈ is C₁-C₄alkyl, or phenyl which can be substituted by C₁-C₄alkyl or halogen, in the presence of a base, or
b) a pyridylsulfonamide of the formula XII 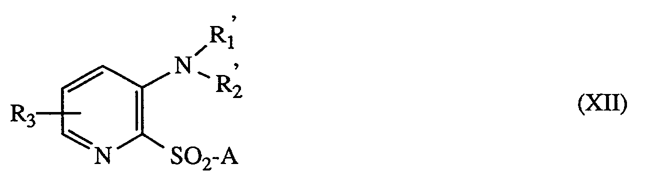 in which R₁', R₂' are as defined above under R₁ and R₂, with the exception of hydrogen, R₃ is as defined under formula I, and A is 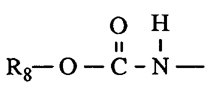 or O=C=N-, R₈ being as defined under formula III, with a 2-aminopyrimidine or -triazine of the formula XI 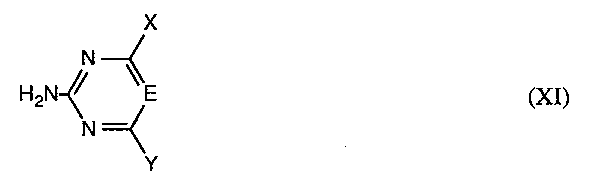 in which E, X and Y are as defined under formula I, in the presence of a base.

2. A process according to claim 1, wherein, in the compounds of the formula I, R₁ and R₂ independently of one another are hydrogen, C₁-C₈alkyl or C₃-C₈cycloalkyl; C₂-C₈alkyl or C₃-C₈cycloalkyl which are monosubstituted or polysubstituted by halogen; C₁-C₈alkyl or C₃-C₈cycloalkyl which are substituted by -NR₅R₆, C₁-C₄alkoxy or C₁-C₄alkyl-S(O)ₙ-; C₁-C₄alkyl or C₃-C₆cycloalkyl which are substituted by C₂-C₄alkenyl, C₅-C₆cycloalkenyl, C₂-C₆alkynyl or C₄-C₈alkadienyl; it being possible for the C₂-C₄alkenyl, C₅-C₆cycloalkenyl and C₄-C₈alkadienyl radicals to be monosubstituted or polysubstituted by halogen; or R₁ and R₂ together are a 4-5-membered C₄-C₅alkylene chain which can be interrupted by oxygen, sulfur or N-R₇.

3. A process according to claim 1, wherein, in the compounds of the formula I, R₁ and R₂ independently of one another are hydrogen, C₁-C₈alkyl or C₃-C₈cycloalkyl; C₂-C₈alkyl or C₃-C₈cycloalkyl which are monosubstituted or polysubstituted by halogen; C₁-C₈alkyl or C₃-C₈cycloalkyl which are substituted by -NR₅R₆, C₁-C₄alkoxy or C₁-C₄alkyl-S(O)ₙ-; C₁-C₄alkyl or C₃-C₆cycloalkyl which are substituted by C₂-C₄alkenyl, C₅-C₆cycloalkenyl, C₂-C₆alkynyl or C₄-C₈alkadienyl; it being possible for the C₂-C₄alkenyl, C₅-C₆cycloalkenyl and C₄-C₈alkadienyl radicals to be monosubstituted or polysubstituted by halogen.

4. A process according to claim 1, wherein, in the compounds of the formula I, at least one of the substituents R₁, R₂ and R₃ is other than hydrogen.

5. A process according to claim 1, wherein, in the compounds of the formula I, R₁ and R₂ independently of one another are hydrogen, C₁-C₆alkyl or C₃-C₆cycloalkyl; C₂-C₆alkyl or C₃-C₆cycloalkyl which are monosubstituted to trisubstituted by halogen; C₁-C₆alkyl or C₃-C₆cycloalkyl which are substituted by -NR₅R₆, C₁-C₄alkoxy or C₁-C₄alkyl-S(O)ₙ-; C₁-C₄alkyl or C₃-C₆cycloalkyl which are substituted by C₂-C₄alkenyl, C₅-C₆cycloalkenyl, C₂-C₄alkynyl or C₄-C₈alkadienyl; it being possible for the C₂-C₄alkenyl, C₅-C₆cycloalkenyl and C₄-C₈alkadienyl radicals to be monosubstituted to trisubstituted by halogen; and R₃ is hydrogen, halogen, C₁-C₄alkyl, C₁-C₄alkoxy or C₁-C₄alkylthio; or C₁-C₄alkyl, C₁-C₄alkoxy or C₁-C₄alkylthio which are monosubstituted to trisubstituted by halogen.

6. A process according to claim 1, wherein, in the compounds of the formula I, X is C₁-C₃alkyl, C₁-C₃alkoxy or C₁-C₃alkoxy which is monosubstituted to trisubstituted by halogen; and
Y is chlorine, C₁-C₃alkyl, C₁-C₃alkoxy or C₁-C₃alkoxy which is monosubstituted to trisubstituted by halogen.

7. A process according to claim 1, wherein, in the compounds of the formula I, X is methyl, methoxy, ethoxy or difluoromethoxy; and Y is methyl, methoxy, ethoxy, difluoromethoxy or chlorine.

8. A process according to claim 1, wherein, in the compounds of the formula I, X is methoxy or ethoxy; and Y is methyl or methoxy.

9. A process according to claim 1, wherein, in the compounds of the formula I, E is the methine bridge.

10. A process according to claim 1, wherein, in the compounds of the formula I, R₃ is in the 6-position of the pyridine ring.

11. A process according to claim 1, wherein, in the compounds of the formula I, R₃ is hydrogen.

12. A process according to claim 1, wherein, in the compounds of the formula I, R₂ and R₃ are hydrogen and R₁ is C₁-C₆alkyl or C₃-C₆cycloalkyl; C₂-C₆alkyl or C₃-C₆cycloalkyl which are monosubstituted to trisubstituted by halogen; C₁-C₆alkyl or C₃-C₆cycloalkyl which are substituted by -NR₅R₆, C₁-C₄alkoxy or C₁-C₄alkyl-S(O)ₙ-; C₁-C₄alkyl or C₃-C₆cycloalkyl which are substituted by C₂-C₄alkenyl, C₅-C₆cycloalkenyl, C₂-C₄alkynyl or C₄-C₈alkadienyl; it being possible for the C₂-C₄alkenyl, C₅-C₆cycloalkenyl and C₄-C₈alkadienyl radicals to be monosubstituted to trisubstituted by halogen.

13. A process according to claim 1, wherein, in the compounds of the formula I, R₁ is C₁-C₆alkyl, allyl or propargyl, and R₂ is hydrogen, methyl or allyl.

14. A process according to claim 1, wherein the compound of the formula I is N-(3-n-Butylaminopyridin-2-ylsulfonyl)-N'-(4,6-dimethoxypyrimidin-2-yl)urea.

15. A process for the preparation of a compound of the formula Ia 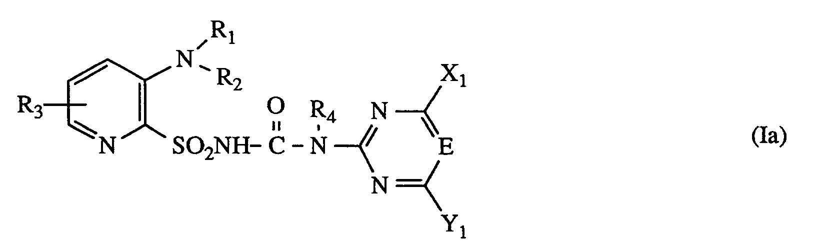 in which X₁ and Y₁ independently of one another are chlorine or difluoromethoxy and R₁, R₂, R₃, R₄ and E are as defined under formula I in claim 1, which comprises reacting a pyridylsulfonamide of the formula II 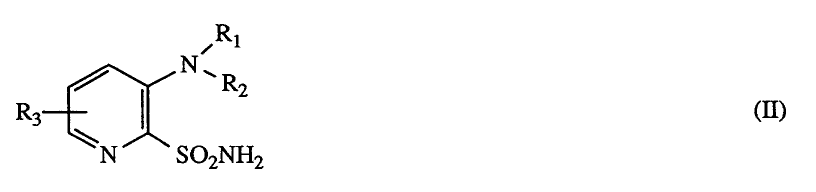 in which R₁, R₂ and R₃ are as defined under formula I in claim 1, with a pyrimidinyl or triazinyl isocyanate of the formula IV 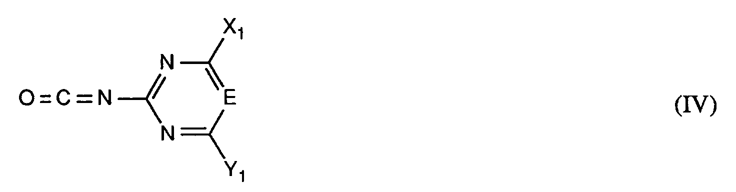 in which X₁ and Y₁ are as defined under formula Ia and E is as defined under formula I, in the presence of a base.

16. A method of controlling undesired plant growth, which comprises applying an effective amount of an active substance of the formula I according to claim 1, or a composition comprising this active substance, to the plants or their environment.

17. A method according to claim 16, in which an amount of active substance of between 0.001 and 2 kg is applied per hectare.

18. A method of inhibiting plant growth, which comprises applying an effective amount of an active substance of the formula I according to claim 1, or a composition comprising this active substance, to the plants or their environment.

19. A method according to claim 16 for selective pre-emergence or post-emergence control of weeds in crops of useful plants.

## Revendications (Revendications pour l'(les) Etat(s) contractant(s) suivant(s): CH, DE, FR, GB, IT, LI)

1. N-pyridinesulfonyl-N'-pyrimidinyl- et - triazinyl-urées de formule I 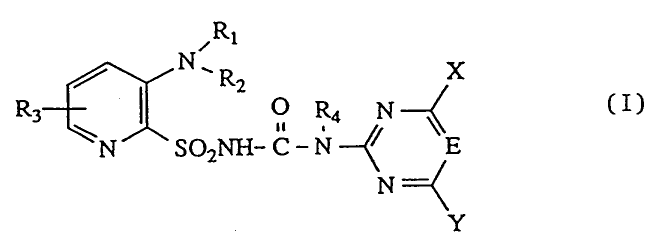 dans laquelle
R₁ et R₂ représentent chacun, indépendamment l'un de l'autre, l'hydrogène, un groupe alkyle en C1-C8 ou cycloalkyle en C3-C8; un groupe alkyle en C2-C8 ou cycloalkyle en C3-C8 portant un ou plusieurs substituants halogéno; un groupe alkyle en C1-C8 ou cycloalkyle en C3-C8 portant un substituant -NR₅R₆, alcoxy en C1-C4 ou (alkyle en C1-C4)-S(O)ₙ; un groupe alkyle en C1-C4 ou cycloalkyle en C3-C6 portant un substituant alcényle en C2-C4, cycloalcényle en C5-C6, alcynyle en C2-C6 ou alcadiényle en C4-C8; les groupes alcényle en C2-C4, cycloalcényle en C5-C6 et alcadiényle en C4-C8 pouvant porter un ou plusieurs substituants halogéno; ou bien R₁ et R₂ forment ensemble une chaîne alkylène en C4-C10 à 4 ou 5 chaînons qui peut être interrompue par l'oxygène, le soufre ou N-R₇;
R₃ représente l'hydrogène, un halogène, un groupe alkyle en C1-C4, alcoxy en C1-C4 ou alkylthio en C1-C4; un groupe alkyle en C1-C4, alcoxy en C1-C4 ou alkylthio en C1-C4 portant un ou plusieurs substituants halogéno;
R₄, R₅, R₆ et R₇ représentent chacun, indépendamment les uns des autres, l'hydrogène ou un groupe alkyle en C1-C4; n est égal à 0, 1 ou 2;
X représente un groupe alkyle en C1-C3, un groupe alkyle en C1-C3 portant un à trois substituants halogéno; un groupe alcoxy en C1-C3 ou alcoxy en C1-C3 portant un à trois substituants halogéno;
Y représente un halogène, un groupe alkyle en C1-C3, alkyle en C1-C3 portant un à trois substituants halogéno; un groupe alcoxy en C1-C3, alcoxy en C1-C3 portant un à trois substituants halogéno; un groupe cyclopropyle, méthylamino ou diméthylamino; et
E représente l'azote ou le groupe méthine,
et les sels de ces composés.

2. Composés de formule I selon revendication 1, caractérisés en ce que
R₁ et R₂ représentent chacun, indépendamment l'un de l'autre, l'hydrogène, un groupe alkyle en C1-C8 ou cycloalkyle en C3-C8; un groupe alkyle en C2-C8 ou cycloalkyle en C3-C8 portant un ou plusieurs substituants halogéno; un groupe alkyle en C1-C8 ou cycloalkyle en C3-C8 portant un substituant -NR₅R₆, alcoxy en C1-C4 ou (alkyle en C1-C4)-S(O)ₙ; un groupe alkyle en C1-C4 ou cycloalkyle en C3-C6 portant un substituant alcényle en C2-C4, cycloalcényle en C5-C6, alcynyle en C2-C6 ou alcadiényle en C4-C8; les groupes alcényle en C2-C4, cycloalcényle en C5-C6 et alcadiényle en C4-C8 pouvant porter un ou plusieurs substituants halogéno; ou bien R₁ et R₂ forment ensemble une chaîne alkylène en C4-C5 à 4 ou 5 chaînons qui peut être interrompue par l'oxygène, le soufre ou N-R₇.

3. Composés de formule I selon revendication 1, caractérisés en ce que
R₁ et R₂ représentent chacun, indépendamment l'un de l'autre, l'hydrogène, un groupe alkyle en C1-C8 ou cycloalkyle en C3-C8; un groupe alkyle en C2-C8 ou cycloalkyle en C3-C8 portant un ou plusieurs substituants halogéno; un groupe alkyle en C1-C8 ou cycloalkyle en C3-C8 portant un substituant -NR₅R₆, alcoxy en C1-C4 ou (alkyle en C1-C4)-S(O)ₙ; un groupe alkyle en C1-C4 ou cycloalkyle en C3-C6 portant un substituant alcényle en C2-C4, cycloalcényle en C5-C6, alcynyle en C2-C6 ou alcadiényle en C4-C8; les groupes alcényle en C2-C4, cycloalcényle en C5-C6 et alcadiényle en C4-C8 pouvant porter un ou plusieurs substituants halogéno.

4. Composés de formule I selon revendication 1, caractérisés en ce que l'un au moins des symboles R₁, R₂ et R₃ a une signification autre que l'hydrogène.

5. Composés de formule I selon revendication 1, caractérisés en ce que
R₁ et R₂ représentent chacun, indépendamment l'un de l'autre, l'hydrogène, un groupe alkyle en C1-C6 ou cycloalkyle en C3-C6; un groupe alkyle en C2-C6 ou cycloalkyle en C3-C6 portant un à trois substituants halogéno; un groupe alkyle en C1-C6 ou cycloalkyle en C3-C6 portant un substituant -NR₅R₆, alcoxy en C1-C4 ou (alkyle en C1-C4)-S(O)ₙ; un groupe alkyle en C1-C4 ou cycloalkyle en C3-C6 portant un substituant alcényle en C2-C4, cycloalcényle en C5-C6, alcynyle en C2-C4 ou alcadiényle en C4-C8; les groupes alcényle en C2-C4, cycloalcényle en C5-C6 et alcadiényle en C4-C8 pouvant porter un à trois substituants halogéno; et
R₃ représente l'hydrogène, un halogène, un groupe alkyle en C1-C4, alcoxy en C1-C4 ou alkylthio en C1-C4; ou un groupe alkyle en C1-C4, alcoxy en C1-C4 ou alkylthio en C1-C4 portant un à trois substituants halogéno.

6. Composés de formule I selon revendication 1, caractérisés en ce que :
X représente un groupe alkyle en C1-C3, alcoxy en C1-C3 ou alcoxy en C1-C3 portant un à trois substituants halogéno; et
Y représente le chlore, un groupe alkyle en C1-C3, alcoxy en C1-C3 ou alcoxy en C1-C3 portant un à trois substituants halogéno.

7. Composés de formule I selon revendication 1, caractérisés en ce que
X représente un groupe méthyle, méthoxy, éthoxy ou difluorométhoxy; et
Y représente un groupe méthyle, méthoxy, éthoxy, difluorométhoxy ou le chlore.

8. Composés de formule I selon revendication 1, caractérisés en ce que X représente un groupe méthoxy ou éthoxy et Y un groupe méthyle ou méthoxy.

9. Composés de formule I selon revendication 1, caractérisés en ce que E représente le pont méthine.

10. Composés de formule I selon revendication 1, caractérisés en ce que R₃ est en position 6 du cycle pyridine.

11. Composés de formule I selon revendication 1, caractérisés en ce que R₃ représente l'hydrogène.

12. Composés de formule I selon revendication 1, caractérisés en ce que
R₂ et R₃ représentent l'hydrogène et R₁ un groupe alkyle en C1-C6 ou cycloalkyle en C3-C6; un groupe alkyle en C2-C6 ou cycloalkyle en C3-C6 portant un à trois substituants halogéno; un groupe alkyle en C1-C6 ou cycloalkyle en C3-C6 portant un substituant -NR₅R₆, alcoxy en C1-C4 ou (alkyle en C1-C4)-S(O)ₙ; un groupe alkyle en C1-C4 ou cycloalkyle en C3-C6 portant un substituant alcényle en C2-C4, cycloalcényle en C5-C6, alcynyle en C2-C4 ou alcadiényle en C4-C8; les groupes alcényle en C2-C4, cycloalcényle en C5-C6 et alcadiényle en C4-C8 pouvant porter un à trois substituants halogéno.

13. Composés de formule I selon revendication 1, caractérisés en ce que R₁ représente un groupe alkyle en C1-C6, allyle ou propargyle et R₂ l'hydrogène, un groupe méthyle ou allyle.

14. La N-(3-n-butylaminopyridine-2-yl-sulfonyl)-N'-(4,6-diméthoxypyrimidine-2-yl)-urée selon revendication 1.

15. Procédé de préparation des composés de formule I de la revendication 1, caractérisé en ce que
a) on fait réagir un pyridylsulfonamide de formule II 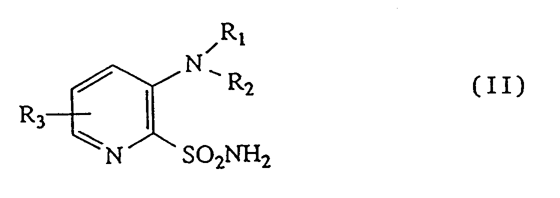 dans laquelle R₁, R₂ et R₃ ont les significations indiquées en référence à la formule I dans la revendication 1, avec un N-pyrimidinyl- ou N-triazinylcarbamate de formule III 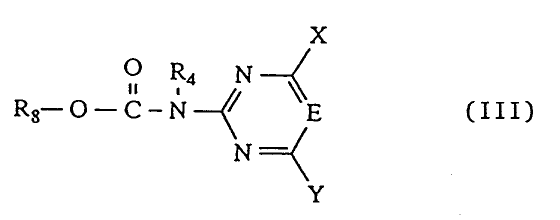 dans laquelle X, Y, R₄ et E ont les significations indiquées en référence à la formule I dans la revendication 1, et R₈ représente un groupe alkyle en C1-C4 ou phényle, lequel peut être substitué par des groupes alkyle en C1-C4 ou des halogènes, en présence d'une base, ou bien
b) on fait réagir un pyridylsulfonamide de formule XII 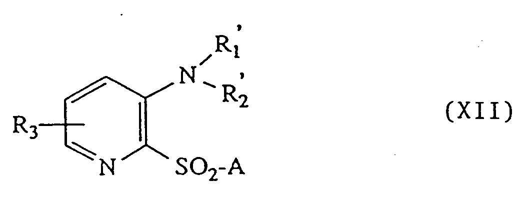 dans laquelle R'₁, R'₂ ont les significations indiquées en référence à R₁ et R₂ dans la formule I de la revendication 1, à l'exception de l'hydrogène, R₃ a les significations indiquées en référence à la formule I dans la revendication 1 et A représente 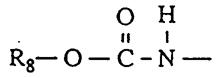 ou O=C=N-, R₈ ayant les significations indiquées en référence à la formule III, en présence d'une base, avec une 2-amino-pyridine ou - triazine de formule XI 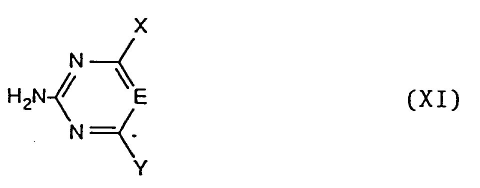 dans laquelle E, X et Y ont les significations indiquées en référence à la formule I.

16. Procédé de préparation des composés de formule Ia 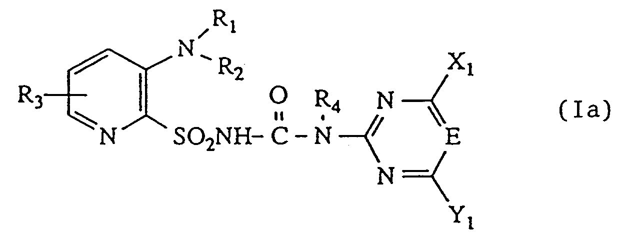 dans laquelle X₁ et Y₁ représentent chacun, indépendamment l'un de l'autre, le chlore ou un groupe difluorométhoxy, et R₁, R₂, R₃, R₄ et E ont les significations indiquées en référence à la formule I dans la revendication 1, caractérisé en ce que l'on fait réagir un pyridylsulfonamide de formule II 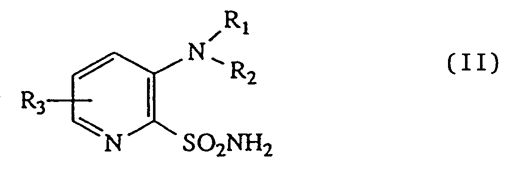 dans laquelle R₁, R₂ et R₃ ont les significations indiquées en référence à la formule I dans la revendication 1, en présence d'une base, avec un pyrimidinyl- ou triazinyl-isocyanate de formule IV 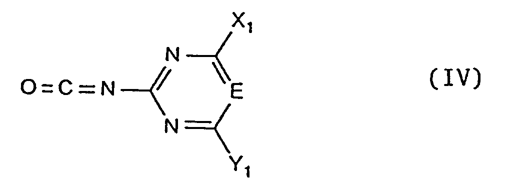 dans laquelle X₁ et Y₁ ont les significations indiquées en référence à la formule la et E les significations indiquées en référence à la formule I.

17. Composés de formule II 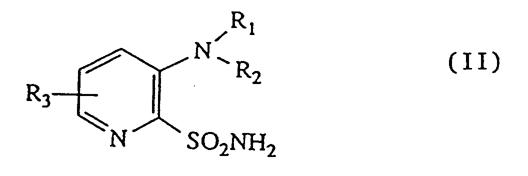 dans laquelle R₁, R₂ et R₃ ont les significations indiquées en référence à la formule I dans la revendication 1, sous réserve que R₁, R₂ et R₃ ne peuvent représenter tous l'hydrogène.

18. Un produit herbicide et inhibiteur de la croissance des végétaux, caractérisé en ce qu'il contient une ou plusieurs sulfonylurées de formule I selon revendication 1.

19. Produit selon revendication 16, caractérisé en ce qu'il contient de 0,1 à 95% d'une substance active de formule I selon revendication 1.

20. Procédé pour combattre les croissances de végétaux indésirables, caractérisé en ce que l'on applique une substance active de formule I selon revendication 1, ou un produit contenant une telle substance active, en quantité efficace, sur les végétaux ou leur habitat.

21. Procédé selon revendication 20, caractérisé en ce que l'on applique une quantité de substance active de 0,001 à 2 kg par ha.

22. Procédé pour inhiber la croissance de végétaux, caractérisé en ce que l'on applique une substance de formule I selon revendication 1, ou un produit contenant une telle substance active, en quantité efficace, sur les végétaux ou leur habitat.

23. Procédé selon revendication 20, pour la lutte sélective en pré-levée ou post-levée contre les végétaux adventices dans les cultures de végétaux utiles.

## Revendications (Revendications pour l'(les) Etat(s) contractant(s) suivant(s): ES)

1. Procédé de préparation des N-pyridinesulfonyl-N'-pyrimidinyl et -triazinyl-urées de formule I 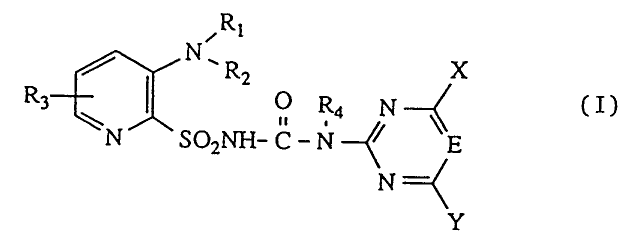 dans laquelle
R₁ et R₂ représentent chacun, indépendamment l'un de l'autre, l'hydrogène, un groupe alkyle en C1-C8 ou cycloalkyle en C3-C8; un groupe alkyle en C2-C8 ou cycloalkyle en C3-C8 portant un ou plusieurs substituants halogéno; un groupe alkyle en C1-C8 ou cycloalkyle en C3-C8 portant un substituant -NR₅R₆, alcoxy en C1-C4 ou (alkyle en C1-C4)-S(O)ₙ; un groupe alkyle en C1-C4 ou cycloalkyle en C3-C6 portant un substituant alcényle en C2-C4, cycloalcényle en C5-C6, alcynyle en C2-C6 ou alcadiényle en C4-C8; les groupes alcényle en C2-C4, cycloalcényle en C5-C6 et alcadiényle en C4-C8 pouvant porter un ou plusieurs substituants halogéno; ou bien R₁ et R₂ forment ensemble une chaîne alkylène en C4-C10 à 4 ou 5 chaînons qui peut être interrompue par l'oxygène, le soufre ou N-R₇;
R₃ représente l'hydrogène, un halogène, un groupe alkyle en C1-C4, alcoxy en C1-C4 ou alkylthio en C1-C4; un groupe alkyle en C1-C4, alcoxy en C1-C4 ou alkylthio en C1-C4 portant un ou plusieurs substituants halogéno;
R₄, R₅, R₆ et R₇ représentant chacun, indépendamment les uns des autres, l'hydrogène ou un groupe alkyle en C1-C4; n est égal à 0, 1 ou 2;
X représente un groupe alkyle en C1-C3, un groupe alkyle en C1-C3 portant un à trois substituants halogéno; un groupe alcoxy en C1-C3 ou alcoxy en C1-C3 portant un à trois substituants halogéno;
Y représente un halogène, un groupe alkyle en C1-C3, alkyle en C1-C3 portant un à trois substituants halogéno; un groupe alcoxy en C1-C3, alcoxy en C1-C3 portant un à trois substituants halogéno; un groupe cyclopropyle, méthylamino ou diméthylamino; et
E représente l'azote ou le groupe méthine.
et des sels de ces composés, caractérisé en ce que
a) on fait réagir un pyridylsulfonamide de formule II 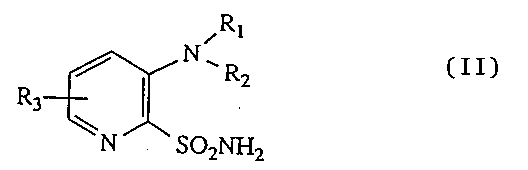 dans laquelle R₁, R₂ et R₃ ont les significations indiquées en référence à la formule I dans la revendication 1, avec un N-pyrimidinyl- ou N-triazinylcarbamate de formule III 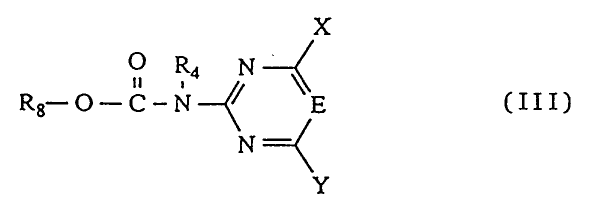 dans laquelle X, Y, R₄ et E ont les significations indiquées en référence à la formule I dans la revendication 1, et R₈ représente un groupe alkyle en C1-C4 ou phényle, lequel peut être substitué par des groupes alkyle en C1-C4 ou des halogènes, en présence d'une base, ou bien
b) on fait réagir un pyridylsulfonamide de formule XII 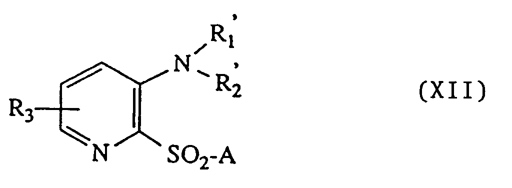 dans laquelle R'₁, R'₂ ont les significations indiquées en référence à R₁ et R₂ dans la formule I de la revendication 1, à l'exception de l'hydrogène, R₃ a les significations indiquées en référence à la formule I dans la revendication 1 et A représente 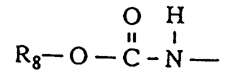 ou O=C=N-, R₈ ayant les significations indiquées en référence à la formule III, en présence d'une base, avec une 2-amino-pyridine ou -triazine de formule XI 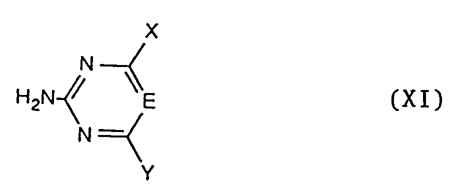 dans laquelle E, X et Y ont les significations indiquées en référence à la formule I.

2. Procédé selon revendication 1, caractérisé en ce que, dans les composés de formule I,
R₁ et R₂ représentent chacun, indépendamment l'un de l'autre, l'hydrogène, un groupe alkyle en C1-C8 ou cycloalkyle en C3-C8; un groupe alkyle en C2-C8 ou cycloalkyle en C3-C8 portant un ou plusieurs substituants halogéno; un groupe alkyle en C1-C8 ou cycloalkyle en C3-C8 portant un substituant -NR₅R₆, alcoxy en C1-C4 ou (alkyle en C1-C4)-S(O)ₙ; un groupe alkyle en C1-C4 ou cycloalkyle en C3-C6 portant un substituant alcényle en C2-C4, cycloalcényle en C5-C6, alcynyle en C2-C6 ou alcadiényle en C4-C8; les groupes alcényle en C2-C4, cycloalcényle en C5-C6 et alcadiényle en C4-C8 pouvant porter un ou plusieurs substituants halogéno; ou bien R₁ et R₂ forment ensemble une chaîne alkylène en C4-C5 à 4 ou 5 chaînons qui peut être interrompue par l'oxygène, le soufre ou N-R₇.

3. Procédé selon revendication 1, caractérisé en ce que, dans les composés de formule I,
R₁ et R₂ représentent chacun, indépendamment l'un de l'autre, l'hydrogène, un groupe alkyle en C1-C8 ou cycloalkyle en C3-C8; un groupe alkyle en C2-C8 ou cycloalkyle en C3-C8 portant un ou plusieurs substituants halogéno; un groupe alkyle en C1-C8 ou cycloalkyle en C3-C8 portant un substituant -NR₅R₆, alcoxy en C1-C4 ou (alkyle en C1-C4)-S(O)ₙ; un groupe alkyle en C1-C4 ou cycloalkyle en C3-C6 portant un substituant alcényle en C2-C4, cycloalcényle en C5-C6, alcynyle en C2-C6 ou alcadiényle en C4-C8; les groupes alcényle en C2-C4, cycloalcényle en C5-C6 et alcadiényle en c4-C8 pouvant porter un ou plusieurs substituants halogéno.

4. Procédé selon revendication 1, caractérisé en ce que, dans les composés de formule I, au moins un des symboles R₁, R₂ et R₃ a une signification autre que l'hydrogène.

5. Procédé selon revendication 1, caractérisé en ce que, dans les composés de formule I,
R₁ et R₂ représentent chacun, indépendamment l'un de l'autre, l'hydrogène, un groupe alkyle en C1-C6 ou cycloalkyle en C3-C6; un groupe alkyle en C2-C6 ou cycloalkyle en C3-C6 portant un à trois substituants halogéno; un groupe alkyle en C1-C6 ou cycloalkyle en C3-C6 portant un substituant -NR₅R₆, alcoxy en C1-C4 ou (alkyle en C1-C4)-S(O)ₙ; un groupe alkyle en C1-C4 ou cycloalkyle en C3-C6 portant un substituant alcényle en C2-C4, cycloalcényle en C5-C6, alcynyle en C2-C4 ou alcadiényle en C4-C8; les groupes alcényle en C2-C4, cycloalcényle en C5-C6 et alcadiényle en C4-C8 pouvant porter un à trois substituants halogéno; et
R₃ représente l'hydrogène, un halogène, un groupe alkyle en C1-C4, alcoxy en C1-C4 ou alkylthio en C1-C4; ou un groupe alkyle en C1-C4, alcoxy en C1-C4 ou alkylthio en C1-C4 portant un à trois substituants halogéno.

6. Procédé selon revendication 1, caractérisé en ce que, dans les composés de formule I
X représente un groupe alkyle en C1-C3, alcoxy en C1-C3 ou alcoxy en C1-C3 portant un à trois substituants halogéno; et
Y représente le chlore, un groupe alkyle en C1-C3, alcoxy en C1-C3 ou alcoxy en C1-C3 portant un à trois substituants halogéno.

7. Procédé selon revendication 1, caractérisé en ce que, dans les composés de formule I
X représente un groupe méthyle, méthoxy, éthoxy ou difluorométhoxy; et
Y représente un groupe méthyle, méthoxy, éthoxy, difluorométhoxy ou le chlore.

8. Procédé selon revendication 1, caractérisé en ce que, dans les composés de formule I, X représente un groupe méthoxy ou éthoxy et Y un groupe méthyle ou méthoxy.

9. Procédé selon revendication 1, caractérisé en ce que, dans les composés de formule I, E représente le pont méthine.

10. Procédé selon revendication 1, caractérisé en ce que, dans les composés de formule I, R₃ est en position 6 du cycle pyridine.

11. Procédé selon revendication 1, caractérisé en ce que, dans les composés de formule I, R₃ représente l'hydrogène.

12. Procédé selon revendication 1, caractérisé en ce que, dans les composés de formule I
R₂ et R₃ représentent l'hydrogène et R₁ un groupe alkyle en C1-C6 ou cycloalkyle en C3-C6; un groupe alkyle en C2-C6 ou cycloalkyle en C3-C6 portant un à trois substituants halogéno; un groupe alkyle en C1-C6 ou cycloalkyle en C3-C6 portant un substituant -NR₅R₆, alcoxy en C1-C4 ou (alkyle en C1-C4)-S(O)ₙ; un groupe alkyle en C1-C4 ou cycloalkyle en C3-C6 portant un substituant alcényle en C2-C4, cycloalcényle en C5-C6, alcynyle en C2-C4 ou alcadiényle en C4-C8; les groupes alcényle en C2-C4, cycloalcényle en C5-C6 et alcadiényle en C4-C8 pouvant porter un à trois substituants halogéno.

13. Procédé selon revendication 1, caractérisé en ce que, dans les composés de formule I,
R₁ représente un groupe alkyle en C1-C6, allyle ou propargyle et R₂ l'hydrogène, un groupe méthyle ou allyle.

14. Procédé selon revendication 1, caractérisé en ce que le composé de formule I est la N-(3-n-butylaminopyridine-2-yl-sulfonyl)-N'-(4,6-diméthoxypyrimidine-2-yl)-urée.

15. Procédé de préparation des composés de formule Ia 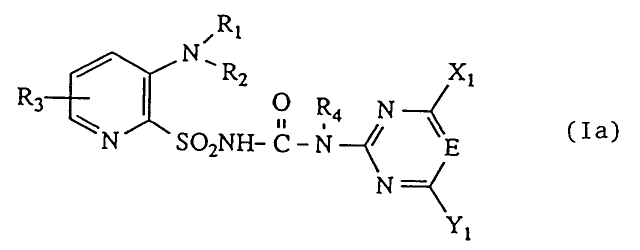 dans laquelle X₁ et Y₁ représentent chacun,indépendamment l'un de l'autre, le chlore ou un groupe difluorométhoxy, et R₁, R₂, R₃, R₄ et E ont les significations indiquées en référence à la formule I dans la revendication 1, caractérisé en ce que l'on fait réagir un pyridylsulfonamide de formule II 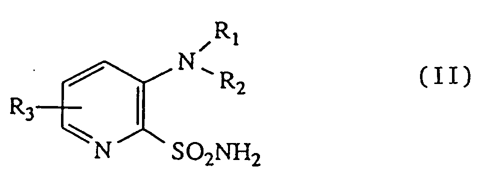 dans laquelle R₁, R₂ et R₃ ont les significations indiquées en référence à la formule I dans la revendication 1, en présence d'une base, avec un pyrimidinyl- ou triazinyl-isocyanate de formule IV 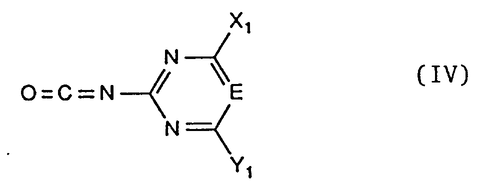 dans laquelle X₁ et Y₁ ont les significations indiquées en référence à la formule Ia et E les significations indiquées en référence à la formule I.

16. Procédé pour combattre les croissances de végétaux indésirables, caractérisé en ce que l'on applique une substance active de formule I selon revendication 1, ou un produit contenant une telle substance active, en quantité efficace, sur les végétaux ou leur habitat.

17. Procédé selon revendication 16, caractérisé en ce que l'on applique une quantité de substance active de 0,001 à 2 kg par ha.

18. Procédé pour inhiber la croissance de végétaux, caractérisé en ce que l'on applique une substance de formule I selon revendication 1, ou un produit contenant une telle substance active, en quantité efficace, sur les végétaux ou leur habitat.

19. Procédé selon revendication 16, pour la lutte sélective en pré-levée ou en post-levée contre les végétaux adventices dans les cultures de végétaux utiles.
